# EUROPEAN PATENT SPECIFICATION

(11) **EP 2 293 818 B1**
(45) Date of publication and mention of the grant of the patent: **28.04.2021**
(21) Application number: 09746991.0
(22) Date of filing: 15.05.2009
(51) Int. Cl.: A61K 47/60

(54) **CONJUGATES OF BUTYRYLCHOLINESTERASE AND A POLYMER**
KONJUGATE AUS BUTYRYLCHOLINESTERASE UND EINES POLYMERS
CONJUGUÉS DE BUTYRYLCHOLINESTÉRASE ET DE POLYMÈRE

(30) Priority: 16.05.2008 US 127928
(43) Date of publication of application: 16.03.2011
(73) Proprietor: Nektar Therapeutics, San Francisco, CA 94158 (US)
(72) Inventor: BOSSARD, Mary, J., Madison AL 35758 (US); ZAPPE, Harold, Harvest AL 35749 (US); LEE, Seoju, Madison AL 35756 (US); FERNANDO, Lal, A. R., Readington NJ 08870 (US)
(74) Representative: Boult Wade Tennant LLP
(86) International application number: PCT/US2009/003035
(87) International publication number: WO 2009/139905

(56) References cited:
- WO-A2-02/087624
- WO-A2-2006/063055
- WO-A2-2006/137052
- WO-A2-2008/019036

## Description

### FIELD OF THE INVENTION

The present invention relates generally to conjugates comprising a cholinesterase moiety and a polymer. In addition, the invention relates to compositions comprising such conjugates and methods for synthesizing such conjugates.

### BACKGROUND OF THE INVENTION

The human nervous system controls bodily functions through the transmission of electrical signals over specialized nerve cells. With respect to the gaps between nerve cells (or between a nerve cell and an effector cell), however, continuation of the signal is typically achieved via chemical means. Chemical transmission through the nerve gap or "synapse" takes place via the release of a substance known as a "neurotransmitter" (alternatively known as a "neuromediator"). Upon release, the neurotransmitter crosses the synapse by diffusion and activates (or inhibits, depending on the system) the postsynaptic cell by binding to a receptor located on the postsynaptic cell. The signal having thus been passed to the postsynaptic cell, enzymes in the synapse degrade the neurotransmitter so as to prevent repeated signal transfer to the postsynaptic cell. In this way, signals within the nervous system are successfully transmitted.

Nerve cells that release acetylcholine as the neurotransmitter are called cholinergic nerves and are located in both the peripheral and central nervous systems in humans. Acetylcholine is involved with the transmission of signals from specialized motor nerves to the skeletal muscle as well as much of the autonomic nervous system, which controls the smooth muscles and glands associated with (for example) respiration, circulation, digestion, sweating and metabolism. In the body, acetylcholine is degraded -- and therefore its effects controlled -- by acetylcholinesterase located in the synaptic cleft. Given the ubiquity of the acetylcholine/acetylcholinesterase system in the central nervous system, the proper balance and functioning of this system is critical to normal functioning and health.

The balance of the acetylcholine/acetylcholinesterase system in the central nervous system can be disrupted through exposure an acetylcholinesterase inhibitor, which results in the accumulation of acetylcholine in the synaptic cleft. This accumulation, in turn, results in continuous signal propagation (typically via persistent depolarization) and concomitant disruption of effective neural transmission. Such a disruption, if allowed to continue, can cause any number of deleterious conditions and -- if severe -- even death.

O-Isopropyl methylphosphonofluoridate (also known as "sarin") and other organophosphates in its class are irreversible cholinesterase inhibitors. These organophosphates inhibit the activity of cholinesterase by covalently binding to a serine residue in the enzyme which forms the site where acetylcholine normally undergoes hydrolysis. Sarin is such a potent and effective inhibitor of cholinesterase enzymes that it has been developed and used in the military context. Other cholinesterase inhibitors have been used as insecticides and pesticides in the agricultural context.

Exposure to cholinesterase inhibitors can be remedied by the administration of cholinesterase itself. By effectively "saturating" the biological system with cholinesterase, overall normal cholinesterase functioning would remain substantially unaffected insomuch as even though some cholinesterase activity would be inhibited by the cholinesterase inhibitor, the presence of excess cholinesterase activity would minimize the effects of cholinesterase inhibitor exposure. Such an approach would be advantageous for accidental exposure to organophosphates as well as in the defense of a military attack in which sarin or similar chemical agent is used. A recombinant version of human butyrylcholinesterase (BChE), a naturally occurring protein, is being developed under the name PROTEXIA® as a pre- and post-exposure therapy for casualties on the battlefield or civilian victims of nerve agent attacks.

One problem associated with administering an excess of molecules having cholinesterase activity is that these protein-based enzymes themselves degrade relatively quickly *in vivo.* PEGylation, or the attachment of a poly(ethylene glycol) derivative to a protein, has been described as a means to prolong a protein's *in vivo* half-life, thereby resulting in prolonged pharmacologic activity. For example, U.S. Patent Application No. 2004/0147002 describes uses of chemically modified cholinesterases for detoxification of organophosphorus compounds. WO2006063055 also describes cholinesterase conjugates.

Notwithstanding these conjugates, however, there remains a need for other conjugates of cholinesterase. The present invention is therefore directed to such conjugates as well as compositions comprising the conjugates and related methods as described herein, which are believed to be new and completely unsuggested by the art.

### SUMMARY OF THE INVENTION

Accordingly, in one or more embodiments of the invention, a di-PEGylated conjugate comprising two poly(ethylene glycol)s covalently attached to a butyrylcholinesterase dimer derived from two separate butyrylcholinesterase monomers, each butyrylcholinesterase monomer having one poly(ethylene glycol) covalently attached to cysteine residue Cys66.

Also provided in accordance with the invention is a pharmaceutical composition comprising a conjugate of the invention and a pharmaceutically acceptable excipient.

In one or more embodiments of the invention, a method for making a diconjugated butyrylcholinesterase dimer conjugate is provided comprising: (a) combining, under conjugation conditions, a reagent composition comprising a plurality of thiol-selective polymeric reagent molecules with a composition comprising a plurality of butyrylcholinesterase dimer molecules, to thereby form a conjugate mixture comprising monoconjugated butyrylcholinesterase dimers and diconjugated butyrylcholinesterase dimers; (b) subjecting the conjugate mixture to reducing conditions to form a reduced mixture comprising reduced unconjugated butyrylcholinesterase monomers and reduced monoconjugated butyrylcholinesterase monomers; (c) separating the reduced monoconjugated butyrylcholinesterase monomers from the reduced mixture to form a composition comprising reduced monoconjugated butyrylcholinesterase monomers; and (d) removing the reducing conditions from the composition comprising reduced monoconjugated butyrylcholinesterase monomers to thereby form a composition of diconjugated butyrylcholinesterase dimers.

### BRIEF DESCRIPTION OF THE DRAWINGS

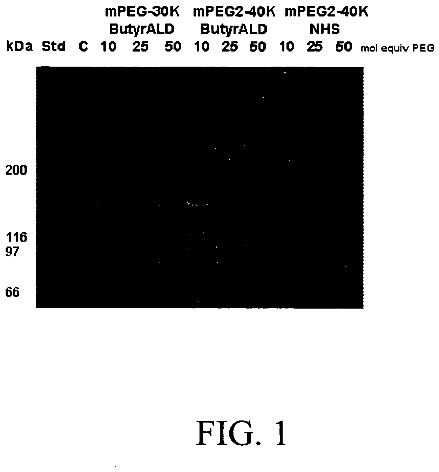

**FIG. 1** depicts an SDS-PAGE analysis of conjugate solutions of rBChE prepared in accordance with Examples 4, 5 and 6 and explained more fully in each of these examples. The lane marked C is the rBChE protein control (not PEGylated). rBChE was PEGylated with different activated PEG regents as indicated above the lanes. Three mol equivalent concentrations (10, 25 and 50) of PEG were tested using the described methods. For each reagent 10 mol equivalents of PEG reagent resulted in low to medium levels of PEGylation, 25 mol equivalents of PEG reagent resulted in medium to high levels of PEGylation and 50 mol equivalents of PEG reagent in very high levels of PEGylation.

### DETAILED DESCRIPTION OF THE INVENTION

It must be noted that, as used in this specification and the intended claims, the singular forms "a," "an," and "the" include plural referents unless the context clearly dictates otherwise. Thus, for example, reference to "a polymer" includes a single polymer as well as two or more of the same or different polymers, reference to "an optional excipient" refers to a single optional excipient as well as two or more of the same or different optional excipients,.

In describing and claiming one or more embodiments of the present invention, the following terminology will be used in accordance with the definitions described below.

"PEG," "polyethylene glycol" and "poly(ethylene glycol)" as used herein, are interchangeable and encompass any nonpeptidic water-soluble poly(ethylene oxide). Typically, PEGs for use in accordance with the invention comprise the following structure "-(OCH₂CH₂)ₙ-" where (n) is 2 to 4000. As used herein, PEG also includes "-CH₂CH₂-O(CH₂CH₂O)ₙ-CH₂CH₂-" and "-(OCH₂CH₂)ₙO-," depending upon whether or not the terminal oxygens have been displaced, e.g., during a synthetic transformation. Throughout the specification and claims, it should be remembered that the term "PEG" includes structures having various terminal or "end capping" groups and so forth. The term "PEG" also means a polymer that contains a majority, that is to say, greater than 50%, of -OCH₂CH₂- repeating subunits. With respect to specific forms, the PEG can take any number of a variety of molecular weights, as well as structures or geometries such as "branched," "linear," "forked," and "multifunctional", to be described in greater detail below.

The terms "end-capped" and "terminally capped" are interchangeably used herein to refer to a terminal or endpoint of a polymer having an end-capping moiety. Typically, although not necessarily, the end-capping moiety comprises a hydroxy or C₁₋₂₀ alkoxy group, more preferably a C₁₋₁₀ alkoxy group, and still more preferably a C₁₋₅ alkoxy group. Thus, examples of end-capping moieties include alkoxy (e.g., methoxy, ethoxy and benzyloxy), as well as aryl, heteroaryl, cyclo and heterocyclo. It must be remembered that the end-capping moiety may include one or more atoms of the terminal monomer in the polymer [e.g., the end-capping moiety "methoxy" in CH₃O(CH₂CH₂O)ₙ- and CH₃(OCH₂CH₂)ₙ-]. In addition, saturated, unsaturated, substituted and unsubstituted forms of each of the foregoing are envisioned. Moreover, the end-capping group can also be a silane. The end-capping group can also advantageously comprise a detectable label. When the polymer has an end-capping group comprising a detectable label, the amount or location of the polymer and/or the moiety (e.g., active agent) to which the polymer is coupled can be determined by using a suitable detector. Such labels include, without limitation, fluorescers, chemiluminescers, moieties used in enzyme labeling, colorimetric (e.g., dyes), metal ions and radioactive moieties. Suitable detectors include photometers, films and spectrometers. The end-capping group can also advantageously comprise a phospholipid. When the polymer has an end-capping group comprising a phospholipid, unique properties are imparted to the polymer and the resulting conjugate. Exemplary phospholipids include, without limitation, those selected from the class of phospholipids called phosphatidylcholines. Specific phospholipids include, without limitation, those selected from the group consisting of dilauroylphosphatidylcholine, dioleylphosphatidylcholine, dipalmitoylphosphatidylcholine, disteroylphosphatidylcholine, behenoylphosphatidylcholine, arachidoylphosphatidylcholine, and lecithin.

"Non-naturally occurring" with respect to a polymer as described herein, means a polymer that in its entirety is not found in nature. A non-naturally occurring polymer may, however, contain one or more monomers or segments of monomers that are naturally occurring, so long as the overall polymer structure is not found in nature.

The term "water soluble" as in a "water-soluble polymer" is any polymer that is soluble in water at room temperature. Typically, a water-soluble polymer will transmit at least about 75%, more preferably at least about 95%, of light transmitted by the same solution after filtering. On a weight basis, a water-soluble polymer will preferably be at least about 35% (by weight) soluble in water, more preferably at least about 50% (by weight) soluble in water, still more preferably about 70% (by weight) soluble in water, and still more preferably about 85% (by weight) soluble in water. It is most preferred, however, that the water-soluble polymer is about 95% (by weight) soluble in water or completely soluble in water.

Molecular weight in the context of a water-soluble polymer, such as PEG, can be expressed as either a number average molecular weight or a weight average molecular weight. Unless otherwise indicated, all references to molecular weight herein refer to the weight average molecular weight. Both molecular weight determinations, number average and weight average, can be measured using gel permeation chromatography or other liquid chromatography techniques. Other methods for measuring molecular weight values can also be used, such as the use of end-group analysis or the measurement of colligative properties (e.g., freezing-point depression, boiling-point elevation, or osmotic pressure) to determine number average molecular weight or the use of light scattering techniques, ultracentrifugation or viscometry to determine weight average molecular weight. The polymers used in the invention are typically polydisperse (i.e., number average molecular weight and weight average molecular weight of the polymers are not equal), possessing low polydispersity values of preferably less than about 1.2, more preferably less than about 1.15, still more preferably less than about 1.10, yet still more preferably less than about 1.05, and most preferably less than about 1.03.

The terms "active," "reactive" or "activated" when used in conjunction with a particular functional group, refers to a reactive functional group that reacts readily with an electrophile or a nucleophile on another molecule. This is in contrast to those groups that require strong catalysts or highly impractical reaction conditions in order to react (i.e., a "nonreactive" or "inert" group).

As used herein, the term "functional group" or any synonym thereof is meant to encompass protected forms thereof as well as unprotected forms.

The terms "spacer moiety," "linkage" and "linker" are used herein to refer to a bond or an atom or a collection of atoms optionally used to link interconnecting moieties such as a terminus of a polymer segment and a cholinesterase moiety or an electrophile or nucleophile of a cholinesterase moiety. The spacer moiety may be hydrolytically stable or may include a physiologically hydrolyzable or enzymatically degradable linkage. Unless the context clearly dictates otherwise, a spacer moiety optionally exists between any two elements of a compound (e.g., the provided conjugates comprising a residue of cholinesterase moiety and water-soluble polymer can attached directly or indirectly through a spacer moiety).

"Alkyl" refers to a hydrocarbon chain, typically ranging from about 1 to 15 atoms in length. Such hydrocarbon chains are preferably but not necessarily saturated and may be branched or straight chain, although typically straight chain is preferred. Exemplary alkyl groups include methyl, ethyl, propyl, butyl, pentyl, 1-methylbutyl, 1-ethylpropyl and 3-methylpentyl. As used herein, "alkyl" includes cycloalkyl as well as cycloalkylene-containing alkyl.

"Lower alkyl" refers to an alkyl group containing from 1 to 6 carbon atoms, and may be straight chain or branched, as exemplified by methyl, ethyl, n-butyl, *i*-butyl, and *t-*butyl.

"Cycloalkyl" refers to a saturated or unsaturated cyclic hydrocarbon chain, including bridged, fused, or spiro cyclic compounds, preferably made up of 3 to about 12 carbon atoms, more preferably 3 to about 8 carbon atoms. "Cycloalkylene" refers to a cycloalkyl group that is inserted into an alkyl chain by bonding of the chain at any two carbons in the cyclic ring system.

"Alkoxy" refers to an -OR group, wherein R is alkyl or substituted alkyl, preferably C₁₋₆ alkyl (e.g., methoxy, ethoxy, propyloxy, and so forth).

The term "substituted" as in, for example, "substituted alkyl," refers to a moiety (e.g., an alkyl group) substituted with one or more noninterfering substituents, such as, but not limited to: alkyl, C₃₋₈ cycloalkyl, e.g., cyclopropyl and cyclobutyl; halo, e.g., fluoro, chloro, bromo, and iodo; cyano; alkoxy, lower phenyl; and substituted phenyl. "Substituted aryl" is aryl having one or more noninterfering groups as a substituent. For substitutions on a phenyl ring, the substituents may be in any orientation (i.e., ortho, meta, or para).

"Noninterfering substituents" are those groups that, when present in a molecule, are typically nonreactive with other functional groups contained within the molecule.

"Aryl" means one or more aromatic rings, each of 5 or 6 core carbon atoms. Aryl includes multiple aryl rings that may be fused, as in naphthyl or unfused, as in biphenyl. Aryl rings may also be fused or unfused with one or more cyclic hydrocarbon, heteroaryl, or heterocyclic rings. As used herein, "aryl" includes heteroaryl.

"Heteroaryl" is an aryl group containing from one to four heteroatoms, preferably sulfur, oxygen, or nitrogen, or a combination thereof. Heteroaryl rings may also be fused with one or more cyclic hydrocarbon, heterocyclic, aryl, or heteroaryl rings.

"Heterocycle" or "heterocyclic" means one or more rings of 5-12 atoms, preferably 5-7 atoms, with or without unsaturation or aromatic character and having at least one ring atom that is not a carbon. Preferred heteroatoms include sulfur, oxygen, and nitrogen.

"Substituted heteroaryl" is heteroaryl having one or more noninterfering groups as substituents.

"Substituted heterocycle" is a heterocycle having one or more side chains formed from noninterfering substituents.

An "organic radical" as used herein shall include alkyl, substituted alkyl, aryl, and substituted aryl.

"Electrophile" and "electrophilic group" refer to an ion or atom or collection of atoms, that may be ionic, having an electrophilic center, i.e., a center that is electron seeking, capable of reacting with a nucleophile.

"Nucleophile" and "nucleophilic group" refers to an ion or atom or collection of atoms that may be ionic having a nucleophilic center, i.e., a center that is seeking an electrophilic center or with an electrophile.

A "physiologically cleavable" or "hydrolyzable" or "degradable" bond is a bond that reacts with water (i.e., is hydrolyzed) under physiological conditions. The tendency of a bond to hydrolyze in water will depend not only on the general type of linkage connecting two central atoms but also on the substituents attached to these central atoms. Appropriate hydrolytically unstable or weak linkages include but are not limited to carboxylate ester, phosphate ester, anhydrides, acetals, ketals, acyloxyalkyl ether, imines, orthoesters, peptides and oligonucleotides.

An "enzymatically degradable linkage" means a linkage that is subject to degradation by one or more enzymes.

A "hydrolytically stable" linkage or bond refers to a chemical bond, typically a covalent bond, that is substantially stable in water, that is to say, does not undergo hydrolysis under physiological conditions to any appreciable extent over an extended period of time. Examples of hydrolytically stable linkages include, but are not limited to, the following: carbon-carbon bonds (e.g., in aliphatic chains), ethers, amides and urethanes. Generally, a hydrolytically stable linkage is one that exhibits a rate of hydrolysis of less than about 1-2% per day under physiological conditions. Hydrolysis rates of representative chemical bonds can be found in most standard chemistry textbooks.

"Pharmaceutically acceptable excipient or carrier" refers to an excipient that may optionally be included in the compositions of the invention and that causes no significant adverse toxicological effects to the patient. "Pharmacologically effective amount," "physiologically effective amount," and "therapeutically effective amount" are used interchangeably herein to mean the amount of a polymer-(cholinesterase) moiety conjugate that is needed to provide a desired level of the conjugate (or corresponding unconjugated cholinesterase moiety) in the bloodstream or in the target tissue. The precise amount will depend upon numerous factors, e.g., the particular cholinesterase moiety, the components and physical characteristics of the therapeutic composition, intended patient population and individual patient considerations, and can readily be determined by one skilled in the art, based upon the information provided herein.

"Multi-functional" means a polymer having three or more functional groups contained therein, where the functional groups may be the same or different. Multi-functional polymeric reagents of the invention will typically contain from about 3-100 functional groups, or from 3-50 functional groups, or from 3-25 functional groups, or from 3-15 functional groups, or from 3 to 10 functional groups, or will contain 3, 4, 5, 6, 7, 8, 9 or 10 functional groups within the polymer backbone.

The term "cholinesterase moiety," as used herein, refers to a moiety having human cholinesterase activity. The cholinesterase moiety will also have at least one electrophilic group or nucleophilic group suitable for reaction with a polymeric reagent. In addition, the term "cholinesterase moiety" encompasses both the cholinesterase moiety prior to conjugation as well as the cholinesterase moiety residue following conjugation. As will be explained in further detail below, one of ordinary skill in the art can determine whether any given moiety has cholinesterase activity. Proteins comprising an amino acid sequence corresponding to any one of SEQ ID NOs: 1 through 2 is a cholinesterase moiety, as well as any protein or polypeptide substantially homologous thereto, that can act as a substrate for a cholinesterase inhibitor. As used herein, the term "cholinesterase moiety" includes such proteins modified deliberately, as for example, by site directed mutagenesis or accidentally through mutations. These terms also include analogs having from 1 to 6 additional glycosylation sites, analogs having at least one additional amino acid at the carboxy terminal end of the protein wherein the additional amino acid(s) includes at least one glycosylation site, and analogs having an amino acid sequence which includes at least one glycosylation site. The term includes both natural and recombinantly produced moieties.

The term "substantially homologous" means that a particular subject sequence, for example, a mutant sequence, varies from a reference sequence by one or more substitutions, deletions, or additions, the net effect of which does not result in an adverse functional dissimilarity between the reference and subject sequences. For purposes of the present invention, sequences having greater than 95 percent homology, equivalent biological properties, and equivalent expression characteristics are considered substantially homologous. For purposes of determining homology, truncation of the mature sequence should be disregarded. Sequences having lesser degrees of identity, comparable bioactivity, and equivalent expression characteristics are considered substantial equivalents. Exemplary cholinesterase moieties for use herein include those sequences that are substantially homologous SEQ ID NO: 1.

The term "fragment" means any protein or polypeptide having the amino acid sequence of a portion or fragment of a cholinesterase moiety, and which has the biological activity of β-cholinesterase. Fragments include proteins or polypeptides produced by proteolytic degradation of a cholinesterase moiety as well as proteins or polypeptides produced by chemical synthesis by methods routine in the art. Enzymatic activity is typically measured, e.g., by enzymatic or inhibitory activity using cultured cell lines or tissue culture based methods.

The term "patient," refers to a living organism suffering from or prone to a condition that can be prevented or treated by administration of an active agent (e.g., conjugate), and includes both humans and animals.

"Optional" or "optionally" means that the subsequently described circumstance may or may not occur, so that the description includes instances where the circumstance occurs and instances where it does not.

"Substantially" means nearly totally or completely, for instance, satisfying one or more of the following: greater than 50%, 51% or greater, 75% or greater, 80% or greater, 90% or greater, and 95% or greater of the condition.

Amino acid residues in peptides are abbreviated as follows: Phenylalanine is Phe or F; Leucine is Leu or L; Isoleucine is Ile or I; Methionine is Met or M; Valine is Val or V; Serine is Ser or S; Proline is Pro or P; Threonine is Thr or T; Alanine is Ala or A; Tyrosine is Tyr or Y; Histidine is His or H; Glutamine is Gln or Q; Asparagine is Asn or N; Lysine is Lys or K; Aspartic Acid is Asp or D; Glutamic Acid is Glu or E; Cysteine is Cys or C; Tryptophan is Trp or W; Arginine is Arg or R; and Glycine is Gly or G.

Turning to one or more embodiments of the invention, a di-PEGylated conjugate comprising two poly(ethylene glycol)s covalently attached to a butyrylcholinesterase dimer derived from two separate butyrylcholinesterase monomers, each butyrylcholinesterase monomer having one poly(ethylene glycol) covalently attached to cysteine residue Cys66 is provided.

### The Cholinesterase Moiety

As previously stated, the conjugate generically comprises a butyrylcholinesterase dimer covalently attached, either directly or through a spacer moiety, to two poly(ethylene glycol)s. As used herein, the term "cholinesterase moiety" shall refer to the cholinesterase moiety prior to conjugation as well as to the cholinesterase moiety following attachment to a nonpeptidic water-soluble polymer. It will be understood, however, that when the original cholinesterase moiety is attached to a nonpeptidic water-soluble polymer, the cholinesterase moiety is slightly altered due to the presence of one or more covalent bonds associated with linkage to the polymer. Often, this slightly altered form of the cholinesterase moiety attached to another molecule is referred to a "residue" of the cholinesterase moiety.

The cholinesterase moiety can be derived from non-recombinant methods and from recombinant methods and the invention is not limited in this regard. In addition, the cholinesterase moiety can be derived from human sources, animal sources, and plant sources.

The cholinesterase moiety can be derived non-recombinantly. For example, it is possible to isolate butyrylcholinesterase from biological systems. As explained in U.S. Patent No. 5,272,080, for example, butyrylcholinesterase can be produced in a purity of at least 90% by subjecting plasma fraction IV-4 alone or in admixture with fraction IV-1 to both anion exchange chromatography and affinity chromatography.

The cholinesterase moiety can be derived from recombinant methods. For example, U.S. Patent Nos. 5,248,604 and 5,595,903 describe recombinant-based methods for producing enzymatically active human cholinesterase. A cholinesterase moiety obtained through the approaches described in these references can be used as a cholinesterase moiety in preparing the conjugates described herein.

The cholinesterase moiety can be expressed in bacterial [e.g., *E. coli,* see, for example, Fischer et al. (1995) Biotechnol. Appl. Biochem. 21(3):295-311], mammalian [see, for example, Kronman et al. (1992) Gene 121:295-304], yeast [e.g., *Pichia pastoris,* see, for example, Morel et al. (1997) Biochem. J. 328(1):121-129], and plant [see, for example, Mor et al. (2001) Biotechnol. Bioeng. 75(3):259-266] expression systems. The expression can occur via exogenous expression (when the host cell naturally contains the desired genetic coding) or via endogenous expression. The production of butyrylcholinesterase in transgenic mammals has been described. See, for example, U.S. Patent Application Publication No. 2004/0016005.

Although recombinant-based methods for preparing proteins can differ, recombinant methods typically involve constructing the nucleic acid encoding the desired polypeptide or fragment, cloning the nucleic acid into an expression vector, transforming a host cell (e.g., plant, bacteria, yeast, transgenic animal cell, or mammalian cell such as Chinese hamster ovary cell or baby hamster kidney cell), and expressing the nucleic acid to produce the desired polypeptide or fragment. Methods for producing and expressing recombinant polypeptides *in vitro* and in prokaryotic and eukaryotic host cells are known to those of ordinary skill in the art.

To facilitate identification and purification of the recombinant polypeptide, nucleic acid sequences that encode for an epitope tag or other affinity binding sequence can be inserted or added in-frame with the coding sequence, thereby producing a fusion protein comprised of the desired polypeptide and a polypeptide suited for binding. Fusion proteins can be identified and purified by first running a mixture containing the fusion protein through an affinity column bearing binding moieties (e.g., antibodies) directed against the epitope tag or other binding sequence in the fusion proteins, thereby binding the fusion protein within the column. Thereafter, the fusion protein can be recovered by washing the column with the appropriate solution (e.g., acid) to release the bound fusion protein. The recombinant polypeptide can also be identified and purified by lysing the host cells, separating the polypeptide, e.g., by size exclusion chromatography, and collecting the polypeptide. These and other methods for identifying and purifying recombinant polypeptides are known to those of ordinary skill in the art. In one or more embodiments of the invention, however, it is preferred that the cholinesterase moiety is not in the form of a fusion protein.

Depending on the system used to express proteins having cholinesterase activity, the cholinesterase moiety can be unglycosylated or glycosylated and either may be used. That is, the cholinesterase moiety can be unglycosylated or the cholinesterase moiety can be glycosylated. In one or more embodiments of the invention, it is preferred that the cholinesterase moiety is glycosylated, preferably at four glycosylation sites. For example, it is also preferred to have the oligosaccharide chain at each glycosylation site terminate in a mannose sugar.

The cholinesterase moiety can advantageously be modified to include and/or substitute one or more amino acid residues such as, for example, lysine, cysteine and/or arginine, in order to provide facile attachment of the polymer to an atom within the side chain of the amino acid. An example of substitution of a cholinesterase moiety is described in Fischer et al. (1995) Biotechnol. Appl. Biochem. 21(3):295-311. In addition, the cholinesterase moiety can be modified to include a non-naturally occurring amino acid residue. Techniques for adding amino acid residues and non-naturally occurring amino acid residues are well known to those of ordinary skill in the art. Reference is made to J. March, Advanced Organic Chemistry: Reactions Mechanisms and Structure, 4th Ed. (New York: Wiley-Interscience, 1992).

In addition, the cholinesterase moiety can advantageously be modified to include attachment of a functional group (other than through addition of a functional group-containing amino acid residue). For example, the cholinesterase moiety can be modified to include a thiol group. In addition, the cholinesterase moiety can be modified to include an N-terminal alpha carbon. In addition, the cholinesterase moiety can be modified to include one or more carbohydrate moieties. In some embodiments of the invention, it is preferred that the cholinesterase moiety is not modified to include a thiol group and/or an N-terminal alpha carbon.

Exemplary cholinesterase moieties are described in the literature and in, for example, US. Patent Application Publication Nos. 2002/0119489, 2006/0263345 and 2008/0213281. Cholinesterase moieties include those having an amino acid sequence comprising sequences selected from the group consisting of SEQ ID NOs: 1 through 2, and sequences substantially homologous thereto. A cholinesterase moiety has the amino acid sequence corresponding to human acetylcholinesterase. Within the invention the cholinesterase moiety is butyrylcholinesterase. A preferred cholinesterase has the amino acid sequence corresponding to human butyrylcholinesterase, e.g., the recombinant version of human butyrylcholinesterase being developed under the PROTEXIA® name (PharmAthene Inc., Annapolis, MD). It is recognized that both acetylcholinesterase and butyrylcholinesterase exist in multiple molecular forms composed of different numbers of catalytic and non-catalytic subunits. In humans, however, both enzymes are composed of subunits of about 600 amino acids each, and both are glycosylated. Acetylcholinesterase may be distinguished from the closely related butyrylcholinesterase by its high specificity for the acetylcholine substrate and sensitivity to selective inhibitors. While acetylcholinesterase is primarily used in the body to hydrolyze acetylcholine, the specific function of butyrylcholinesterase is not as clear. In any event, the terms "acetylcholinesterase" and "butyrylcholinesterase" encompass all of the molecular forms within each enzyme. In some instances, the cholinesterase moiety is in a "monomer" form, wherein a single expression of the corresponding peptide is organized into a discrete unit. Within the invention, the cholinesterase moiety will be in the form of a "dimer" (e.g., a dimer of recombinant human butyrylcholinesterase) wherein two monomer forms of the protein are associated (e.g., by disulfide bonding) to each other. For example, in the context of a dimer of recombinant human butyrylcholinesterase, the dimer may be in the form of two monomers associated to each other by a disulfide bond formed from each monomer's Cys571 residue.

In addition, precursor forms of a protein that has cholinesterase activity can be used.

Truncated versions, hybrid variants, and peptide mimetics of any of the foregoing sequences can also serve as the cholinesterase moiety. Biologically active fragments, deletion variants, substitution variants or addition variants of any of the foregoing that maintain at least some degree of cholinesterase activity can also serve as a cholinesterase moiety.

For any given peptide or protein moiety, it is possible to determine whether that moiety has cholinesterase activity. Various methods for *in vitro* cholinesterase enzymatic activity assays are described in the art. See, for example, Lockridge et al. (1978) J. Biol. Chem. 253:361-366, Lockridge et al. (1997) Biochemistry 36:786-795, Plattborze et al. (2000) Biotechnol. Appl. Biochem. 31:226-229, and Blong et al. (1997) Biochem. J. 327:747-757. Samples can be tested for the presence of enzymatically active cholinesterase activity by using the activity assay of Ellman [Ellman et al. (1961) Biochem. Pharmacol. 7:88]. Levels of cholinesterase activity can be estimated by staining non-denaturing 4-30% polyacrylamide gradient gels with 2 mM echothiophate iodide as substrate (as described in Lockridge et al., *supra),* where this method is a modification of the same assays using 2 mM butrylythiocholine as substrate [from Karnovsky et al. (1964) J. Histochem. Cytochem. 12:219]. Using these methods, the catalytic properties of a moiety of interest, including Km, Vmax, and kcat values, can be determined using butyrylthiocholine or acetylthiocholine as substrate. Other methodologies known in the art can also be used to assess cholinesterase function, including electrometry, spectrophotometry, chromatography, and radiometric methodologies.

### The Water-Soluble Polymer

As previously discussed, each conjugate comprises two poly(ethylene glycol)s covalently attached to a butyrylcholinesterase dimer. A water-soluble polymer is nonpeptidic, nontoxic, non-naturally occurring and biocompatible. With respect to biocompatibility, a substance is considered biocompatible if the beneficial effects associated with use of the substance alone or with another substance (e.g., an active agent such as an cholinesterase moiety) in connection with living tissues (e.g., administration to a patient) outweighs any deleterious effects as evaluated by a clinician, e.g., a physician. With respect to non-immunogenicity, a substance is considered non-immunogenic if the intended use of the substance *in vivo* does not produce an undesired immune response (e.g., the formation of antibodies) or, if an immune response is produced, that such a response is not deemed clinically significant or important as evaluated by a clinician. It is particularly preferred that the nonpeptidic water-soluble polymer is biocompatible and non-immunogenic.

Further, the polymer is typically characterized as having from 2 to about 300 termini. In accordance with the invention the polymers may be polyethylene glycol ("PEG"). Other polymers include poly(propylene glycol) ("PPG"), copolymers of ethylene glycol and propylene glycol, poly(oxyethylated polyol), poly(olefinic alcohol), poly(vinylpyrrolidone), poly(hydroxyalkylmethacrylamide), poly(hydroxyalkylmethacrylate), poly(saccharides), poly(α-hydroxy acid), poly(vinyl alcohol), polyphosphazene, polyoxazolines ("POZ") (which are described in WO 2008/106186), poly(N-acryloylmorpholine), and combinations of any of the foregoing.

The water-soluble polymer is not limited to a particular structure and can be linear (e.g., an end capped, e.g., alkoxy PEG or a bifunctional PEG), branched or multi-armed (e.g., forked PEG or PEG attached to a polyol core), a dendritic (or star) architecture, each with or without one or more degradable linkages.

Typically, activated PEG and other activated water-soluble polymers (i.e., polymeric reagents) are activated with a suitable activating group appropriate for coupling to a desired site on the cholinesterase moiety. Thus, a polymeric reagent will possess a reactive group for reaction with the cholinesterase moiety. Representative polymeric reagents and methods for conjugating these polymers to an active moiety are known in the art and further described in Zalipsky, S., et al., "Use of Functionalized Poly(Ethylene Glycols) for Modification of Polypeptides" in Polyethylene Glycol Chemistry: Biotechnical and Biomedical Applications, J. M. Harris, Plenus Press, New York (1992), and in Zalipsky (1995) Advanced Drug Reviews 16:157-182. Exemplary activating groups suitable for coupling to a cholinesterase moiety include hydroxyl, maleimide, ester, acetal, ketal, amine, carboxyl, aldehyde, aldehyde hydrate, ketone, vinyl ketone, thione, thiol, vinyl sulfone, and hydrazine.

Typically, the weight-average molecular weight of the water-soluble polymer in the conjugate is from about 100 Daltons to about 150,000 Daltons. Exemplary ranges, however, include weight-average molecular weights in the range of greater than 5,000 Daltons to about 100,000 Daltons, in the range of from about 6,000 Daltons to about 90,000 Daltons, in the range of from about 10,000 Daltons to about 85,000 Daltons, in the range of greater than 10,000 Daltons to about 85,000 Daltons, in the range of from about 20,000 Daltons to about 85,000 Daltons, in the range of from about 53,000 Daltons to about 85,000 Daltons, in the range of from about 25,000 Daltons to about 120,000 Daltons, in the range of from about 29,000 Daltons to about 120,000 Daltons, in the range of from about 35,000 Daltons to about 120,000 Daltons, and in the range of from about 40,000 Daltons to about 120,000 Daltons. For any given water-soluble polymer, PEGs having a molecular weight in one or more of these ranges are preferred.

Exemplary weight-average molecular weights for the water-soluble polymer include about 100 Daltons, about 200 Daltons, about 300 Daltons, about 400 Daltons, about 500 Daltons, about 600 Daltons, about 700 Daltons, about 750 Daltons, about 800 Daltons, about 900 Daltons, about 1,000 Daltons, about 1,500 Daltons, about 2,000 Daltons, about 2,200 Daltons, about 2,500 Daltons, about 3,000 Daltons, about 4,000 Daltons, about 4,400 Daltons, about 4,500 Daltons, about 5,000 Daltons, about 5,500 Daltons, about 6,000 Daltons, about 7,000 Daltons, about 7,500 Daltons, about 8,000 Daltons, about 9,000 Daltons, about 10,000 Daltons, about 11,000 Daltons, about 12,000 Daltons, about 13,000 Daltons, about 14,000 Daltons, about 15,000 Daltons, about 20,000 Daltons, about 22,500 Daltons, about 25,000 Daltons, about 30,000 Daltons, about 35,000 Daltons, about 40,000 Daltons, about 45,000 Daltons, about 50,000 Daltons, about 55,000 Daltons, about 60,000 Daltons, about 65,000 Daltons, about 70,000 Daltons, and about 75,000 Daltons. Branched versions of the water-soluble polymer (e.g., a branched 40,000 Dalton water-soluble polymer comprised of two 20,000 Dalton polymers) having a total molecular weight of any of the foregoing can also be used. In one or more embodiments, the conjugate will not have any PEG moieties attached, either directly or indirectly, with a PEG having a weight average molecular weight of less than about 6,000 Daltons.

When used as the polymer in accordance with the invention, PEGs will typically comprise a number of (OCH₂CH₂) monomers [or (CH₂CH₂O) monomers, depending on how the PEG is defined]. As used throughout the description, the number of repeating units is identified by the subscript "*n*" in "(OCH₂CH₂)ₙ." Thus, the value of (n) typically falls within one or more of the following ranges: from 2 to about 3400, from about 100 to about 2300, from about 100 to about 2270, from about 136 to about 2050, from about 225 to about 1930, from about 450 to about 1930, from about 1200 to about 1930, from about 568 to about 2727, from about 660 to about 2730, from about 795 to about 2730, from about 795 to about 2730, from about 909 to about 2730, and from about 1,200 to about 1,900. For any given polymer in which the molecular weight is known, it is possible to determine the number of repeating units (i.e., "n") by dividing the total weight-average molecular weight of the polymer by the molecular weight of the repeating monomer.

One particularly preferred polymer for use in the invention is an end-capped polymer, that is, a polymer having at least one terminus capped with a relatively inert group, such as a lower C₁₋₆ alkoxy group, although a hydroxyl group can also be used. When the polymer is PEG, for example and in accordance with the claims, it is preferred to use a methoxy-PEG (commonly referred to as mPEG), which is a linear form of PEG wherein one terminus of the polymer is a methoxy (-OCH₃) group, while the other terminus is a hydroxyl or other functional group that can be optionally chemically modified.

In one form useful in one or more embodiments of the present invention, free or unbound PEG is a linear polymer terminated at each end with hydroxyl groups:

HO-CH₂CH₂O-(CH₂CH₂O)ₙ-CH₂CH₂-OH,

wherein (n) typically ranges from zero to about 4,000.

The above polymer, alpha-, omega-dihydroxylpoly(ethylene glycol), can be represented in brief form as HO-PEG-OH where it is understood that the -PEG- symbol can represent the following structural unit:

-CH₂CH₂O-(CH₂CH₂O)ₙ-CH₂CH₂-,

wherein (n) is as defined as above.

Another type of PEG useful in one or more embodiments of the present invention is methoxy-PEG-OH, or mPEG in brief, in which one terminus is the relatively inert methoxy group, while the other terminus is a hydroxyl group. The structure of mPEG is given below.

CH₃O-CH₂CH₂O-(CH₂CH₂O)ₙ-CH₂CH₂-OH

wherein (n) is as described above.

Multi-armed or branched PEG molecules, such as those described in U.S. Patent No. 5,932,462, can also be used as the PEG polymer. For example, PEG can have the structure: 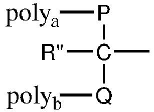 wherein:
polyₐ and poly_{b} are PEG backbones (either the same or different), such as methoxy poly(ethylene glycol);
R" is a nonreactive moiety, such as H, methyl or a PEG backbone; and
P and Q are nonreactive linkages. In a preferred embodiment, the branched PEG polymer is methoxy poly(ethylene glycol) disubstituted lysine. Depending on the specific cholinesterase moiety used, the reactive ester functional group of the disubstituted lysine may be further modified to form a functional group suitable for reaction with the target group within the cholinesterase moiety.

In addition, the PEG can comprise a forked PEG. An example of a forked PEG is represented by the following structure: 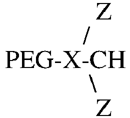 wherein: X is a spacer moiety of one or more atoms and each Z is an activated terminal group linked to CH by a chain of atoms of defined length. International Patent Application Publication WO 99/45964 discloses various forked PEG structures capable of use in one or more embodiments of the present invention. The chain of atoms linking the Z functional groups to the branching carbon atom serve as a tethering group and may comprise, for example, alkyl chains, ether chains, ester chains, amide chains and combinations thereof.

The PEG polymer may comprise a pendant PEG molecule having reactive groups, such as carboxyl, covalently attached along the length of the PEG rather than at the end of the PEG chain. The pendant reactive groups can be attached to the PEG directly or through a spacer moiety, such as an alkylene group.

In addition to the above-described forms of PEG, the polymer can also be prepared with one or more weak or degradable linkages in the polymer, including any of the above-described polymers. For example, PEG can be prepared with ester linkages in the polymer that are subject to hydrolysis. As shown below, this hydrolysis results in cleavage of the polymer into fragments of lower molecular weight:

-PEG-CO₂-PEG- + H₂O → -PEG-CO₂H + HO-PEG-

Other hydrolytically degradable linkages, useful as a degradable linkage within a polymer backbone and/or as a degradable linkage to a cholinesterase moiety, include: carbonate linkages; imine linkages resulting, for example, from reaction of an amine and an aldehyde (see, e.g., Ouchi et al. (1997) Polymer Preprints 38(1):582-3); phosphate ester linkages formed, for example, by reacting an alcohol with a phosphate group; hydrazone linkages which are typically formed by reaction of a hydrazide and an aldehyde; acetal linkages that are typically formed by reaction between an aldehyde and an alcohol; orthoester linkages that are, for example, formed by reaction between a formate and an alcohol; amide linkages formed by an amine group, e.g., at an end of a polymer such as PEG, and a carboxyl group of another PEG chain; urethane linkages formed from reaction of, e.g., a PEG with a terminal isocyanate group and a PEG alcohol; peptide linkages formed by an amine group, e.g., at an end of a polymer such as PEG, and a carboxyl group of a peptide; and oligonucleotide linkages formed by, for example, a phosphoramidite group, e.g., at the end of a polymer, and a 5' hydroxyl group of an oligonucleotide.

Such optional features of the conjugate, i.e., the introduction of one or more degradable linkages into the polymer chain or to the cholinesterase moiety, may provide for additional control over the final desired pharmacological properties of the conjugate upon administration. For example, a large and relatively inert conjugate (i.e., having one or more high molecular weight PEG chains attached thereto, for example, one or more PEG chains having a molecular weight greater than about 10,000, wherein the conjugate possesses essentially no bioactivity) may be administered, which is hydrolyzed to generate a bioactive conjugate possessing a portion of the original PEG chain. In this way, the properties of the conjugate can be more effectively tailored to balance the bioactivity of the conjugate over time.

The water-soluble polymer associated with the conjugate can also be "cleavable." That is, the water-soluble polymer cleaves (either through hydrolysis, enzymatic processes, or otherwise), thereby resulting in the unconjugated cholinesterase moiety. In some instances, cleavable polymers detach from the cholinesterase moiety *in vivo* without leaving any fragment of the water-soluble polymer. In other instances, cleavable polymers detach from the cholinesterase moiety *in vivo* leaving a relatively small fragment (e.g., a succinate tag) from the water-soluble polymer. An exemplary cleavable polymer includes one that attaches to the cholinesterase moiety via a carbonate linkage.

As used herein, the term "polymeric reagent" generally refers to an entire molecule, which can comprise a water-soluble polymer segment and a functional group.

As described above, a conjugate of the invention comprises two poly(ethylene glycol)s covalently attached to a butyrylcholinesterase dimer.

The particular linkage within the moiety having cholinesterase activity and the polymer depends on a number of factors. Such factors include, for example, the particular linkage chemistry employed, the particular cholinesterase moiety, the available functional groups within the cholinesterase moiety (either for attachment to a polymer or conversion to a suitable attachment site) and the presence of additional reactive functional groups within the cholinesterase moiety.

The conjugates of the invention can be, although not necessarily, prodrugs, meaning that the linkage between the polymer and the cholinesterase moiety is hydrolytically degradable to allow release of the parent moiety. Exemplary degradable linkages include carboxylate ester, phosphate ester, thiolester, anhydrides, acetals, ketals, acyloxyalkyl ether, imines, orthoesters, peptides and oligonucleotides. Such linkages can be readily prepared by appropriate modification of either the cholinesterase moiety (e.g., the carboxyl group C terminus of the protein, or a side chain hydroxyl group of an amino acid such as serine or threonine contained within the protein, or a similar functionality within the carbohydrate) and/or the polymeric reagent using coupling methods commonly employed in the art. Most preferred, however, are hydrolyzable linkages that are readily formed by reaction of a suitably activated polymer with a non-modified functional group contained within the moiety having cholinesterase activity.

Alternatively, a hydrolytically stable linkage, such as an amide, urethane (also known as carbamate), amine, thioether (also known as sulfide), or urea (also known as carbamide) linkage can also be employed as the linkage for coupling the cholinesterase moiety. Again, a preferred hydrolytically stable linkage is an amide. In one approach, a water-soluble polymer bearing an activated ester can be reacted with an amine group on the cholinesterase moiety to thereby result in an amide linkage.

The conjugates (as opposed to an unconjugated cholinesterase moiety) may or may not possess a measurable degree of cholinesterase activity. That is to say, a polymer-cholinesterase moiety conjugate in accordance with the invention will possesses anywhere from about 0.1% to about 100% of the bioactivity of the unmodified parent cholinesterase moiety. In some instances, the polymer-cholinesterase moiety conjugates may have greater than 100% bioactivity of the unmodified parent cholinesterase moiety. Preferably, conjugates possessing little or no cholinesterase activity contain a hydrolyzable linkage connecting the polymer to the moiety, so that regardless of the lack (or relatively lack) of activity in the conjugate, the active parent molecule (or a derivative thereof) is released upon aqueous-induced cleavage of the hydrolyzable linkage. Such activity may be determined using a suitable *in-vivo* or *in-vitro* model, depending upon the known activity of the particular moiety having cholinesterase activity employed.

For conjugates possessing a hydrolytically stable linkage that couples the moiety having cholinesterase activity to the polymer, the conjugate will typically possess a measurable degree of bioactivity. For instance, such conjugates are typically characterized as having a bioactivity satisfying one or more of the following percentages relative to that of the unconjugated cholinesterase moiety: at least about 2%, at least about 5%, at least about 10%, at least about 15%, at least about 25%, at least about 30%, at least about 40%, at least about 50%, at least about 60%, at least about 80%, at least about 85%, at least about 90%, at least about 95%, at least about 97%, at least about 100%, and more than 105% (when measured in a suitable model, such as those well known in the art). Preferably, conjugates having a hydrolytically stable linkage (e.g., an amide linkage) will possess at least some degree of the bioactivity of the unmodified parent moiety having cholinesterase activity.

Typically, a protein is expected to share (at least in part) a similar amino acid sequence as the sequence provided in SEQ ID NO: 1 or SEQ ID NO 2. Thus, while reference will be made to specific locations or atoms within SEQ ID NOS: 1 or 2, such a reference is for convenience only and one having ordinary skill in the art will be able to readily determine the corresponding location or atom in other moieties having cholinesterase activity. In particular, the description provided herein for native human cholinesterase is often applicable to fragments, deletion variants, substitution variants or addition variants of any of the foregoing.

It is described that amino groups on cholinesterase moieties could provide a point of attachment between the cholinesterase moiety and the water-soluble polymer. Using the amino acid sequence provided in SEQ ID NOs: 1 through 2, it is evident that there are several lysine residues in each having an ε-amino acid that may be available for conjugation. Further, the N-terminal amine of any protein can also serve as a point of attachment.

There are a number of examples of suitable polymeric reagents useful for forming covalent linkages with available amines of a cholinesterase moiety. Specific examples, along with the corresponding conjugate, are provided in Table 1, below. In the table, the variable (n) represents the number of repeating monomeric units and "-NH-(ChE)" represents the residue of the cholinesterase moiety following conjugation to the polymeric reagent. While each polymeric portion [e.g., (OCH₂CH₂)ₙ or (CH₂CH₂O)ₙ] presented in Table 1 terminates in a "CH₃" group, other groups (such as H and benzyl) can be substituted therefor.

**Table 1**

| Amine-Selective Polymeric Reagents and the Cholinesterase Moiety Conjugate Formed Therefrom | |
|---|---|
| Polymeric Reagent | Corresponding Conjugate |
| 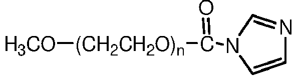 | 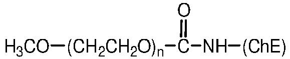 |
| mPEG-Oxycarbonylimidazole Reagents | Carbamate Linkage |
| 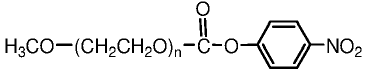 | 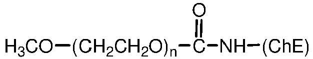 |
| mPEG Nitrophenyl Reagents | Carbamate Linkage |
| 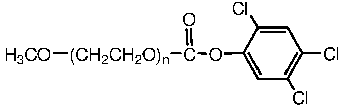 | 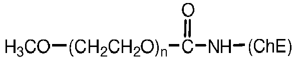 |
| mPEG-Trichlorophenyl Carbonate Reagents | Carbamate Linkage |
| 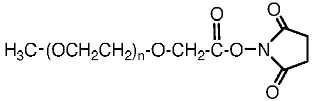 | 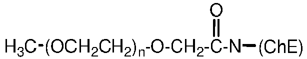 |
| mPEG-Succinimidyl Reagents | Amide Linkage |
| 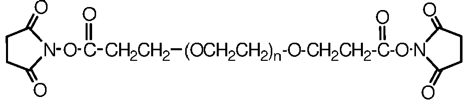 | 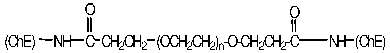 |
| Homobifunctional PEG-Succinimidyl Reagents | Amide Linkages |
| 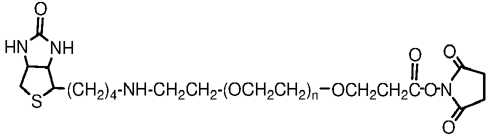 | 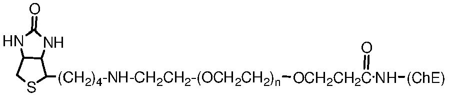 |
| Heterobifunctional PEG-Succinimidyl Reagents | Amide Linkage |
| 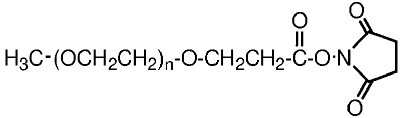 | 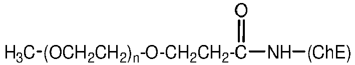 |
| mPEG-Succinimidyl Reagents | Amide Linkage |
| 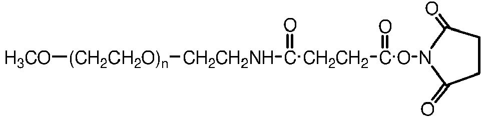 | 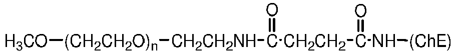 |
| mPEG-Succinimdyl Reagents | Amide Linkage |
| 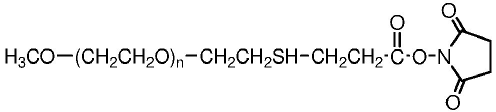 | 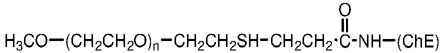 |
| mPEG Succinimidyl Reagents | Amide Linkage |
| 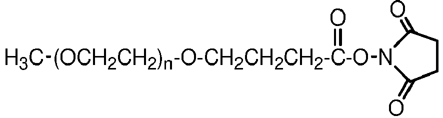 | 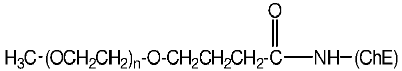 |
| mPEG-Succinimidyl Reagents | Amide Linkage |
| 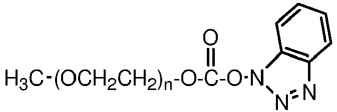 | 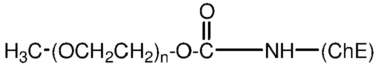 |
| mPEG-Benzotriazole Carbonate Reagents | Carbamate Linkage |
| 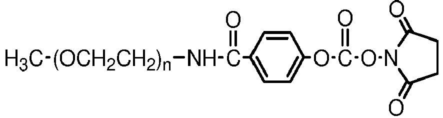 | 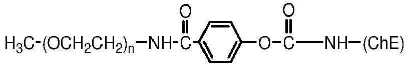 |
| mPEG-Succinimidyl Reagents | Carbamate Linkage |
| 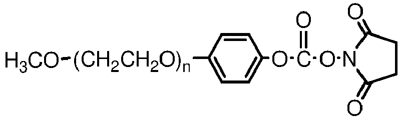 | 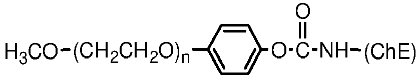 |
| mPEG-Succinimidyl Reagents | Amide Linkage |
| 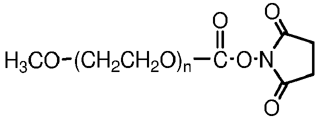 | 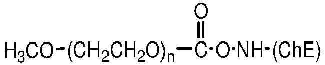 |
| mPEG Succinimidyl Reagents | Amide Linkage |
| 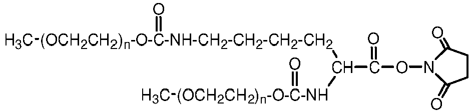 | 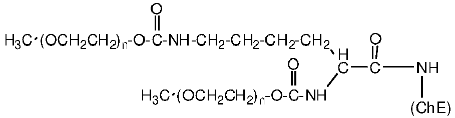 |
| Branched mPEG2-N-Hydroxysuccinimide Reagents | Amide Linkage |
| 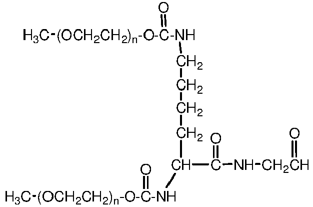 | 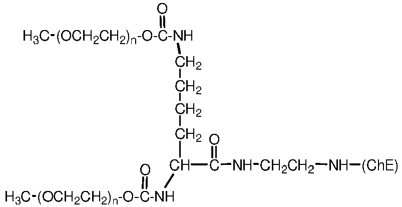 |
| Branched mPEG2-Aldehyde Reagents | Secondary Amine Linkage |
| 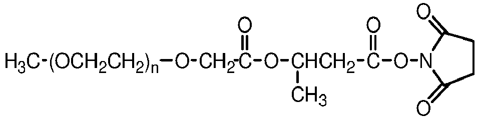 | 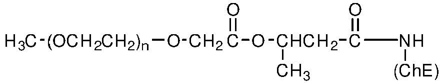 |
| mPEG-Succinimidyl Reagents | Amide Linkage |
| 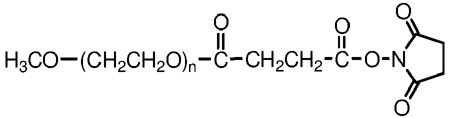 | 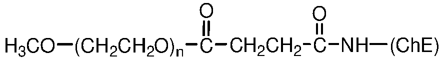 |
| mPEG-Succinimidyl Reagents | Amide Linkage |
| 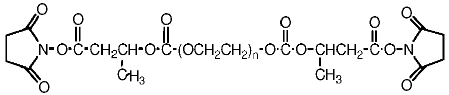 | 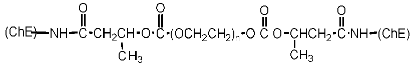 |
| Homobifunctional PEG-Succinimidyl Reagents | Amide Linkages |
| 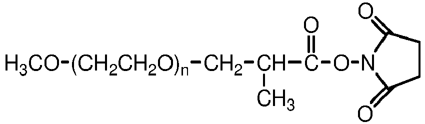 | 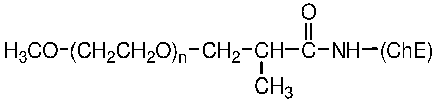 |
| mPEG-Succinimidyl Reagents | Amide Linkage |
| 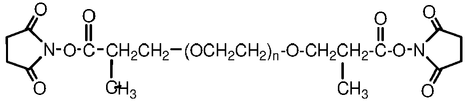 | 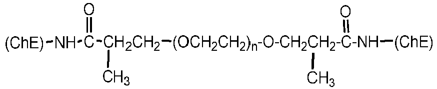 |
| Homobifunctional PEG-Succinimidyl Propionate Reagents | Amide Linkages |
| 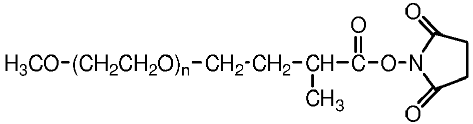 | 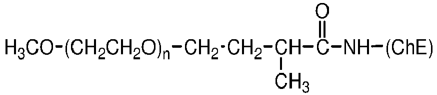 |
| mPEG-Succinimidyl Reagents | Amide Linkage |
| 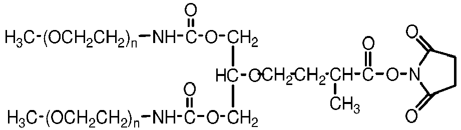 | 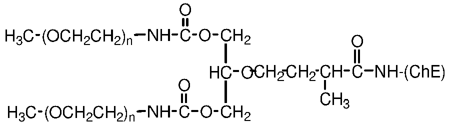 |
| Branched mPEG2-N-Hydroxysuccinimide Reagents | Amide Linkage |
| 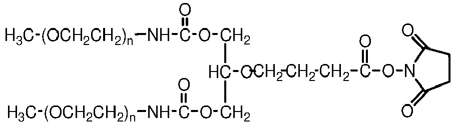 | 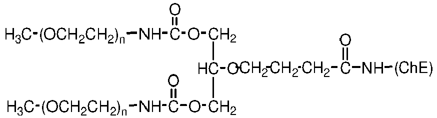 |
| Branched mPEG2-N-Hydroxysuccinimide Reagents | Amide Linkage |

| Polymeric Reagent | Corresponding Conjugate |
|---|---|
| 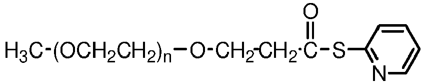 | 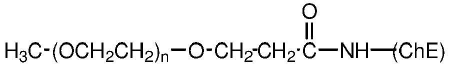 |
| mPEG-Thioester Reagents | Amide Linkage (typically to cholinesterase moiety having an N-terminal cysteine or histidine) |
| 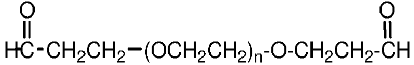 | 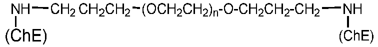 |
| Homobifunctional PEG Propionaldehyde Reagents | Secondary Amine Linkages |
| 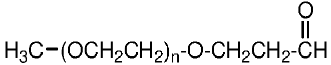 | H₃C-(OCH₂CH₂)ₙ-O-CH₂CH₂-CH₂-NH-((ChE) |
| mPEG Propionaldehyde Reagents | Secondary Amine Linkage |
| 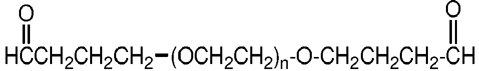 | 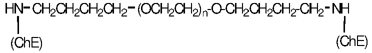 |
| Homobifunctional PEG Butyraldehyde Reagents | Secondary Amine Linkages |
| 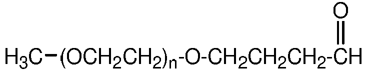 | H₃C-(OCH₂CH₂)ₙ-O-CH₂CH₂-CH₂-CH₂-NH-(ChE) |
| mPEG Butryaldehyde Reagents | Secondary Amine Linkage |
| 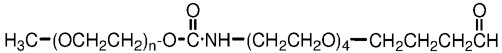 | 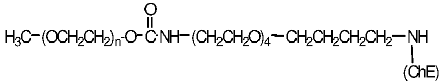 |
| mPEG Butryaldehyde Reagents | Secondary Amine Linkage |
| 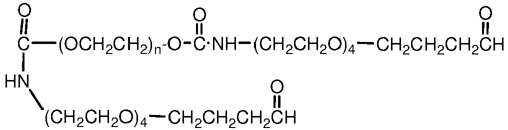 | 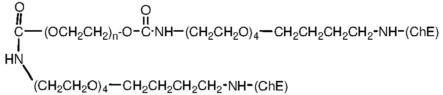 |
| Homobifunctional PEG Butryaldehyde Reagents | Secondary Amine Linkages |
| 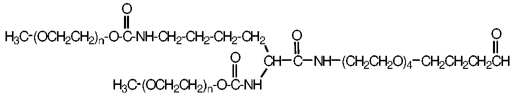 | 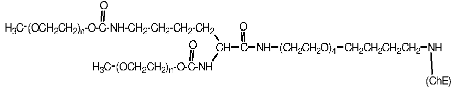 |
| Branched mPEG2 Butyraldehyde Reagents | Secondary Amine Linkage |
| 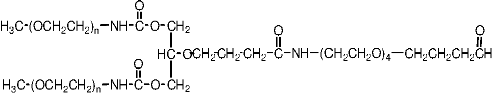 | 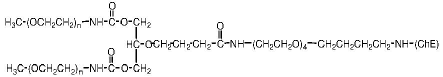 |
| Branched mPEG2 Butyraldehyde Reagents | Secondary Amine Linkage |
| 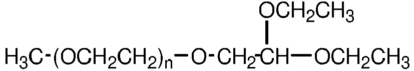 | 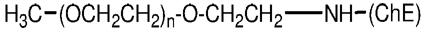 |
| mPEG Acetal Reagents | Secondary Amine Linkage |
| 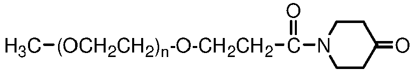 | 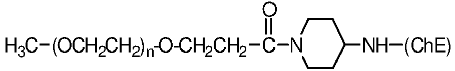 |
| mPEG Piperidone Reagents | Secondary Amine Linkage (to a secondary carbon) |
| 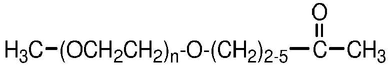 | 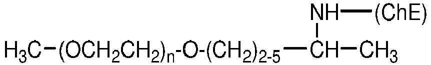 |
| mPEG Methylketone Reagents | secondary amine linkage (to a secondary carbon) |
| 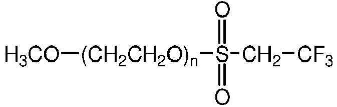 | 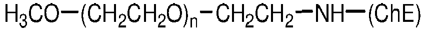 |
| mPEG Tresylate Reagents | Secondary Amine Linkage |
| 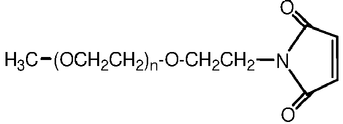 | 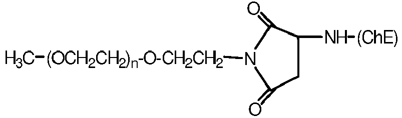 |
| mPEG Maleimide Reagents (under certain reaction conditions such as pH > 8) | Secondary Amine Linkage |
| 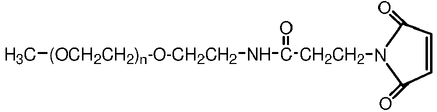 | 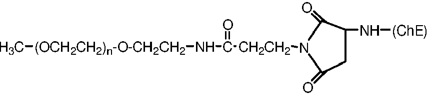 |
| mPEG Maleimide Reagents (under certain reaction conditions such as pH > 8) | Secondary Amine Linkage |
| 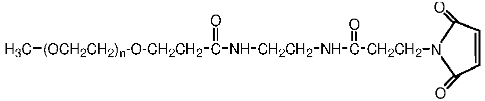 | 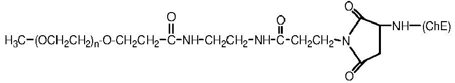 |
| mPEG Maleimide Reagents (under certain reaction conditions such as pH > 8) | Secondary Amine Linkage |
| 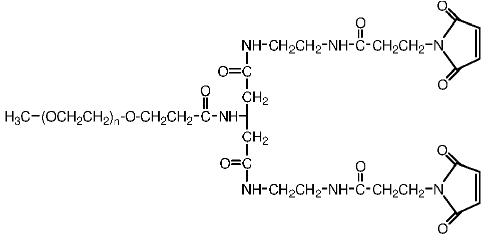 | 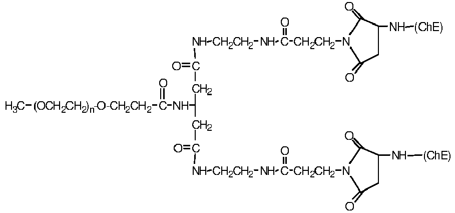 |
| mPEG Forked Maleimide Reagents (under certain reaction conditions such as pH > 8) | Secondary Amine Linkages |
| 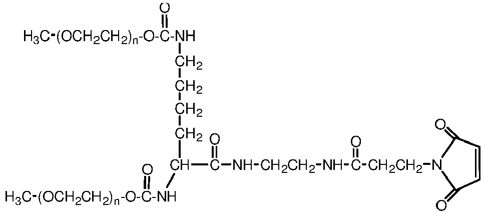 | 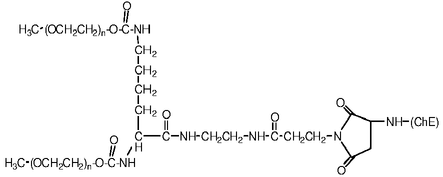 |
| branched mPEG2 Maleimide Reagents (under certain reaction conditions such as pH > 8) | Secondary Amine Linkage |
| 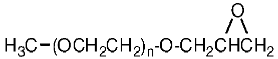 | 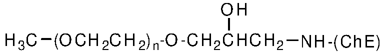 |
| mPEG Epoxide Reagents (under certain reaction conditions such as pH > 8) | Secondary Amine Linkage |

Conjugation of a polymeric reagent to an amino group of a cholinesterase moiety can be accomplished by a variety of techniques. In one approach, a cholinesterase moiety can be conjugated to a polymeric reagent functionalized with a succinimidyl derivative (or other activated ester group, wherein approaches similar to those described for these alternative activated ester group-containing polymeric reagents can be used). In this approach, the polymer bearing a succinimidyl derivative can be attached to the cholinesterase moiety in an aqueous media at a pH of 7 to 9.0, although using different reaction conditions (e.g., a lower pH such as 6 to 7, or different temperatures and/or less than 15 °C) can result in the attachment of the polymer to a different location on the cholinesterase moiety. In addition, an amide linkage can be formed by reacting an amine-terminated nonpeptidic, water-soluble polymer with a cholinesterase moiety bearing an activating a carboxylic acid group.

A described conjugate comprises the following structure 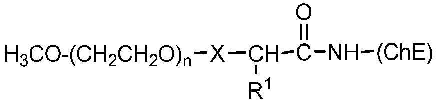 wherein:
(n) is an integer having a value of from 2 to 4000;
X is a spacer moiety;
R¹ is an organic radical; and
ChE is a residue of a cholinesterase moiety.

Another described conjugate comprises the following structure: 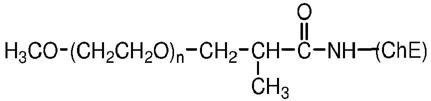 wherein (n) an integer having a value of from 2 to 4000 and ChE is a residue of a cholinesterase moiety.

Typical of another approach useful for conjugating the cholinesterase moiety to a polymeric reagent is use of reductive amination to conjugate a primary amine of a cholinesterase moiety with a polymeric reagent functionalized with a ketone, aldehyde or a hydrated form thereof (e.g., ketone hydrate, aldehyde hydrate). In this approach, the primary amine from the cholinesterase moiety reacts with the carbonyl group of the aldehyde or ketone (or the corresponding hydroxyl-containing group of a hydrated aldehyde or ketone), thereby forming a Schiff base. The Schiff base, in turn, can then be reductively converted to a stable conjugate through use of a reducing agent such as sodium borohydride. Selective reactions (e.g., at the N-terminus) are possible, particularly with a polymer functionalized with a ketone or an alpha-methyl branched aldehyde and/or under specific reaction conditions (e.g., reduced pH).

Exemplary conjugates of the invention wherein the water-soluble polymer is in a branched form include those wherein the water-soluble polymer comprises the following structure: 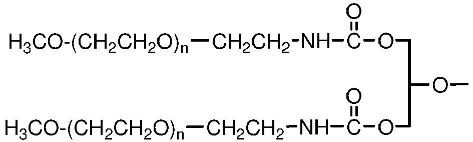 wherein each (n) is independently an integer having a value of from 2 to 4000.

Conjugates comprise the following structure: 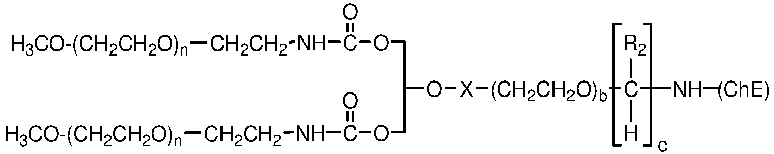 wherein:
each (n) is independently an integer having a value of from 2 to 4000;
X is spacer moiety;
(b) is an integer having a value 2 through 6;
(c) is an integer having a value 2 through 6;
R², in each occurrence, is independently H or lower alkyl; and
ChE is a residue of a cholinesterase moiety.

A conjugate comprises the following structure: 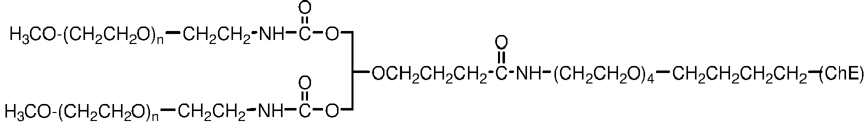 wherein:
each (n) is independently an integer having a value of from 2 to 4000; and
ChE is a residue of a cholinesterase moiety.

Another conjugate comprises the following structure: 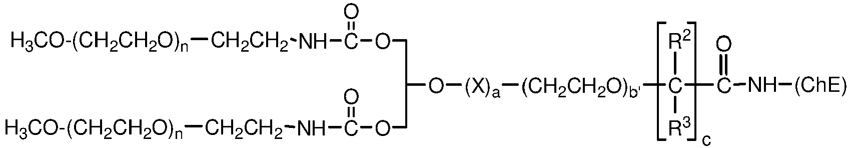 wherein:
each (n) is independently an integer having a value of from 2 to 4000;
(a) is either zero or one;
X, when present, is a spacer moiety comprised of one or more atoms;
(b') is zero or an integer having a value of one through ten;
(c) is an integer having a value of one through ten;
R², in each occurrence, is independently H or an organic radical;
R³, in each occurrence, is independently H or an organic radical; and
ChE is a residue of a cholinesterase moiety.

A conjugate comprises the following structure: 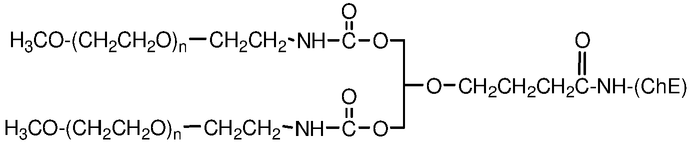 wherein:
each (n) is independently an integer having a value of from 2 to 4000; and
ChE is a residue of cholinesterase moiety.

Carboxyl groups represent another functional group that can serve as a point of attachment on the cholinesterase moiety. Structurally, the conjugate will comprise the following: 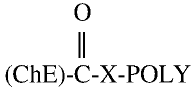 where (ChE) and the adjacent carbonyl group corresponds to the carboxyl-containing cholinesterase moiety, X is a linkage, such as a heteroatom selected from O, N(H), and S, and POLY is a water-soluble polymer such as PEG, optionally terminating in an end-capping moiety.

The C(O)-X linkage results from the reaction between a polymeric derivative bearing a terminal functional group and a carboxyl-containing cholinesterase moiety. As discussed above, the specific linkage will depend on the type of functional group utilized. If the polymer is end-functionalized or "activated" with a hydroxyl group, the resulting linkage will be a carboxylic acid ester and X will be O. If the polymer backbone is functionalized with a thiol group, the resulting linkage will be a thioester and X will be S. When certain multi-arm, branched or forked polymers are employed, the C(O)X moiety, and in particular the X moiety, may be relatively more complex and may include a longer linkage structure.

Water-soluble derivatives containing a hydrazide moiety are also useful for conjugation at a carbonyl and carboxylic acid. To the extent that the cholinesterase moiety does not contain a carbonyl moiety or a carboxylic acid, one can be added using techniques known to one of ordinary skill in the art. For example, a carbonyl moiety can be introduced by reducing a carboxylic acid (e.g., the C-terminal carboxylic acid) and/or by providing glycosylated or glycated (wherein the added sugars have a carbonyl moiety) versions of the cholinesterase moiety. With respect to cholinesterase moieties containing a carboxylic acid, a PEG-hydrazine reagent can, in the presence of a coupling agent (e.g., DCC), covalently attach to the cholinesterase moiety [e.g., mPEG-OCH₂C(O)NHNH₂ + HOC(O)-(ChE) results in mPEG-OCH₂C(O)NHNHC(O)-ChE]. Specific examples of water-soluble derivatives containing a hydrazide moiety, along with the corresponding conjugates, are provided in Table 2, below. In addition, any water-soluble derivative containing an activated ester (e.g., a succinimidyl group) can be converted to contain a hydrazide moiety by reacting the water-soluble polymer derivative containing the activated ester with hydrazine (NH2-NH2) or tert-butyl carbazate [NH₂NHCO₂C(CH₃)₃]. In the table, the variable (n) represents the number of repeating monomeric units and "=C-(ChE)" represents the residue of the cholinesterase moiety following conjugation to the polymeric reagent. Optionally, the hydrazone linkage can be reduced using a suitable reducing agent. While each polymeric portion [e.g., (OCH₂CH₂)ₙ or (CH₂CH₂O)ₙ] presented in Table 2 terminates in a "CH₃" group, other groups (such as H and benzyl) can be substituted therefor.

**Table 2**

| Carboxyl-Specific Polymeric Reagents and the Cholinesterase Moiety Conjugate Formed Therefrom | |
|---|---|
| Polymeric Reagent | Corresponding Conjugate |
| 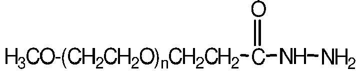 | 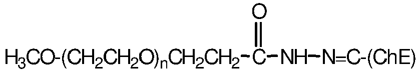 |
| mPEG-Hydrazine Reagents | Hydrazone Linkage |
| 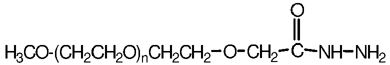 | 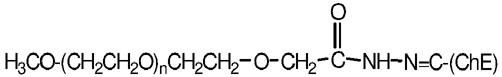 |
| mPEG-Hydrazine Reagents | Hydrazone Linkage |
| 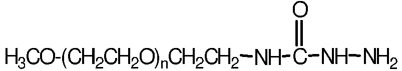 | 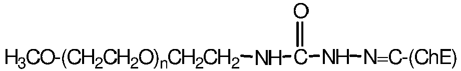 |
| mPEG-Hydrazine Reagents | Hydrazone Linkage |
| 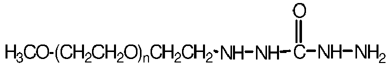 | 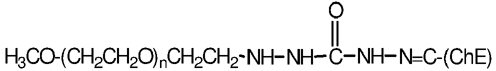 |
| mPEG-Hydrazine Reagents | Hydrazone Linkage |
| 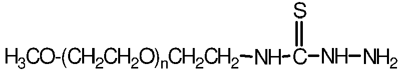 | 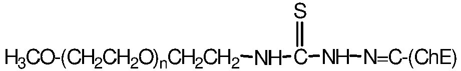 |
| mPEG-Hydrazine Reagents | Hydrazone Linkage |
| 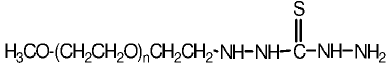 | 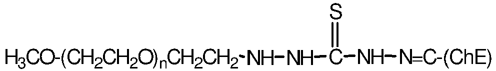 |
| mPEG-Hydrazine Reagents | Hydrazone Linkage |
| 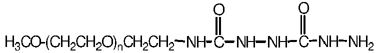 | 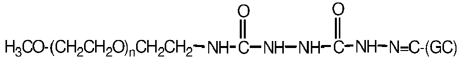 |
| mPEG-Hydrazine Reagents | Hydrazone Linkage |
| 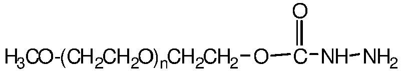 | 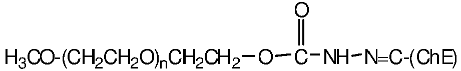 |
| mPEG-Hydrazine Reagents | Hydrazone Linkage |
| 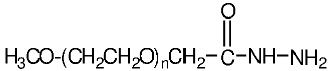 | 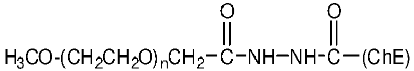 |
| mPEG-Hydrazine Reagents | C(O)NHNHC(O) Linkage |

Thiol groups contained within the cholinesterase moiety can serve as effective sites of attachment for the water-soluble polymer, and within the invention the polymer is attached at Cys66. In particular, cysteine residues provide thiol groups when the cholinesterase moiety is a protein. The thiol groups in such cysteine residues can then be reacted with an activated PEG that is specific for reaction with thiol groups, e.g., an N-maleimidyl polymer or other derivative as described in U.S. Patent No. 5,739,208 and in WO 01/62827. In addition, a protected thiol may be incorporated into an oligosaccharide side chain of an activated glycoprotein, followed by deprotection with a thiol-reactive water-soluble polymer.

Specific examples of reagents, along with the corresponding conjugate, are provided in Table 3, below. In the table, the variable (n) represents the number of repeating monomeric units and "-S-(ChE)" represents the cholinesterase moiety residue following conjugation to the water-soluble polymer. While each polymeric portion [e.g., (OCH₂CH₂)ₙ or (CH₂CH₂O)ₙ] presented in Table 3 terminates in a "CH3" group, other groups (such as H and benzyl) can be substituted therefor.

With respect to SEQ ID NOs: 1 through 2 corresponding to exemplary cholinesterase moieties, it can be seen that there are many thiol-containing cysteine residues. Thus, thiol attachment sites are associated with one of these seven cysteine residues. Although it is preferred not to disrupt any disulfide bonds, it may be possible to attach a polymer within the side chain of one or more of these cysteine residues and retain a degree of activity. Within the invention the location of attachment of a water-soluble polymer is at the thiol-containing cysteine residue corresponding to Cys66 of SEQ ID NO: 2. In addition, it is possible to add a cysteine residue to the cholinesterase moiety using conventional synthetic techniques. See, for example, the procedure described in WO 90/12874 for adding cysteine residues, wherein such procedure can be adapted for a cholinesterase moiety. In addition, conventional genetic engineering processes can also be used to introduce a cysteine residue into the cholinesterase moiety.

**Table 3**

| Thiol-Selective Polymeric Reagents and the Cholinesterase Moiety Conjugate Formed Therefrom | |
|---|---|
| Polymeric Reagent | Corresponding Conjugate |
| 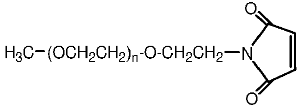 | 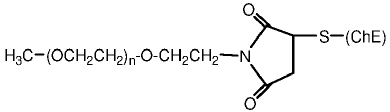 |
| mPEG Maleimide Reagent | Thioether Linkage |
| 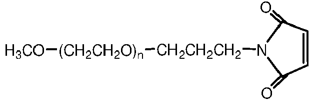 | 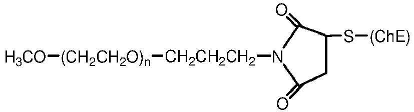 |
| mPEG Maleimide Reagent | Thioether Linkage |
| 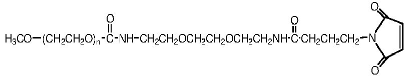 | 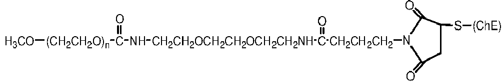 |
| mPEG Maleimide Reagent | Thioether Linkage |
| 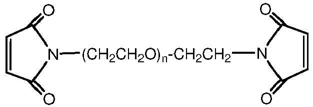 | 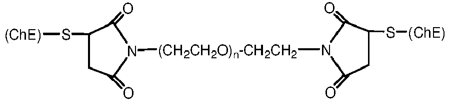 |
| Homobifunctional mPEG Maleimide Reagent | Thioether Linkages |
| 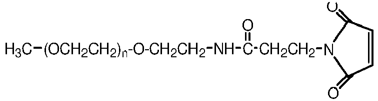 | 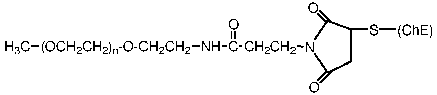 |
| mPEG Maleimide Reagent | Thioether Linkage |
| 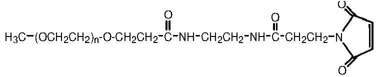 | 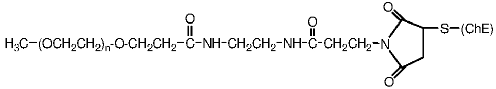 |
| mPEG Maleimide Reagent | Thioether Linkage |
| 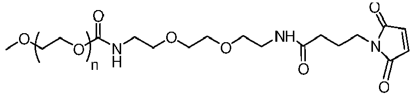 | 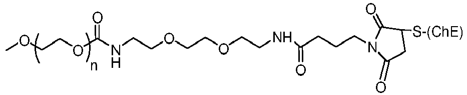 |
| mPEG Maleimide Reagent | Thioether Linkage |
| 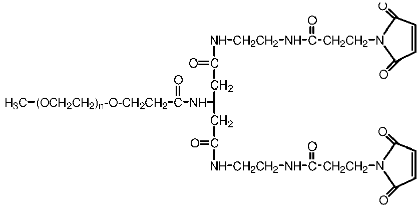 | 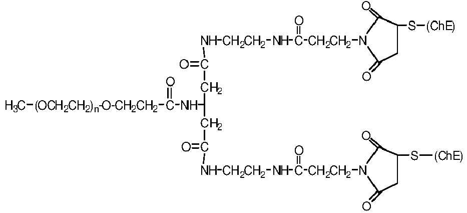 |
| mPEG Forked Maleimide Reagent | Thioether Linkage |
| 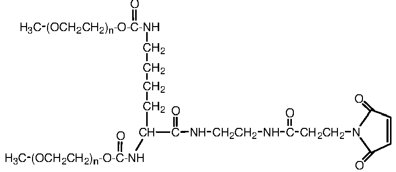 | 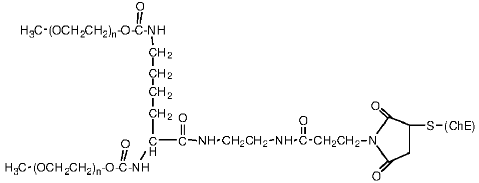 |
| branched mPEG2 Maleimide Reagent | Thioether Linkage |
| 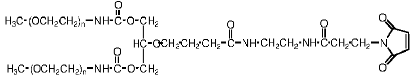 | 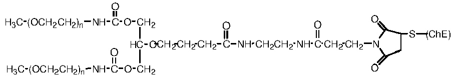 |
| branched mPEG2 Maleimide Reagent | Thioether Linkage |
| 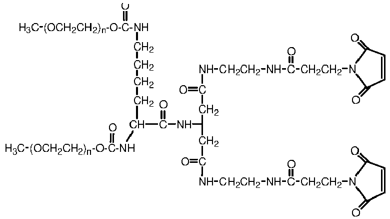 | 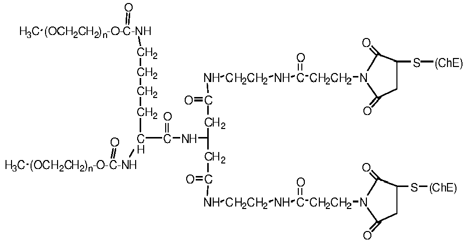 |
| Branched mPEG2 Forked Maleimide Reagent | Thioether Linkages |
| 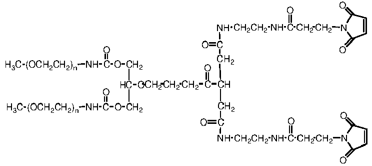 | 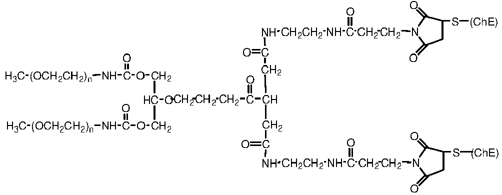 |
| Branched mPEG2 Forked Maleimide Reagent | Thioether Linkages |
| 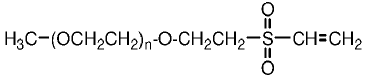 | 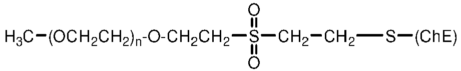 |
| mPEG Vinyl Sulfone Reagent | Thioether Linkage |
| 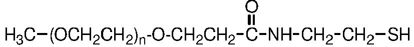 | 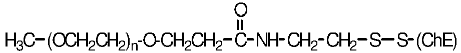 |
| mPEG Thiol Reagent | Disulfide Linkage |
| 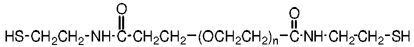 | 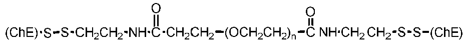 |
| Homobifunctional PEG Thiol Reagent | Disulfide Linkages |
| 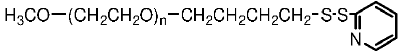 | H₃CO-(CH₂CH₂O)ₙ-CH₂CH₂CH₂CH₂-S-S-(ChE) |
| mPEG Disulfide Reagent | Disulfide Linkage |
| 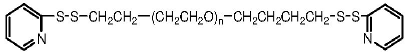 | (ChE)-S-S-CH₂CH₂-(CH₂CH₂-(CH₂CH₂O)ₙ-CH₂CH₂CH₂CH₂-S-S-(ChE) |
| Homobifunctional Disulfide Reagent | Disulfide Linkages |

With respect to conjugates formed from water-soluble polymers bearing one or more maleimide functional groups (regardless of whether the maleimide reacts with an amine or thiol group on the cholinesterase moiety), the corresponding maleamic acid form(s) of the water-soluble polymer can also react with the cholinesterase moiety. Under certain conditions (e.g., a pH of about 7-9 and in the presence of water), the maleimide ring will "open" to form the corresponding maleamic acid. The maleamic acid, in turn, can react with an amine or thiol group of a cholinesterase moiety. Exemplary maleamic acid-based reactions are schematically shown below. POLY represents the water-soluble polymer, and (ChE) represents the cholinesterase moiety.

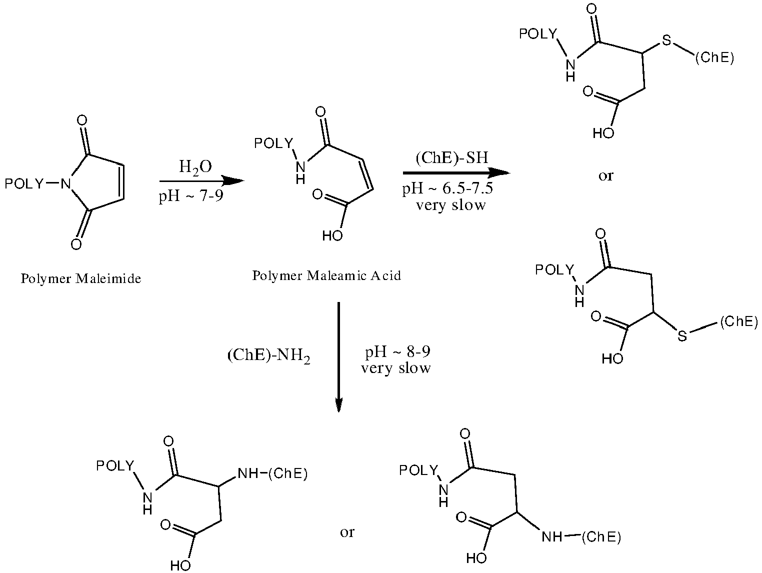

A representative conjugate in accordance with the invention can have the following structure:

POLY-L_{0,1}-C(O)Z-Y-S-S-(ChE)

wherein POLY is a water-soluble polymer, L is an optional linker, Z is a heteroatom selected from the group consisting of O, NH, and S, and Y is selected from the group consisting of C₂₋₁₀ alkyl, C₂₋₁₀ substituted alkyl, aryl, and substituted aryl, and (ChE) is a cholinesterase moiety. Polymeric reagents that can be reacted with a cholinesterase moiety and result in this type of conjugate are described in U.S. Patent Application Publication No. 2005/0014903.

As previously indicated, exemplary conjugates of the invention wherein the water-soluble polymer is in a branched form, will have the branched form of the water-soluble polymer comprise the following structure: 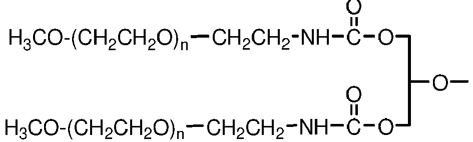 wherein each (n) is independently an integer having a value of from 2 to 4000.

Exemplary conjugates having a water-soluble polymer in branched form are prepared using the following reagent: 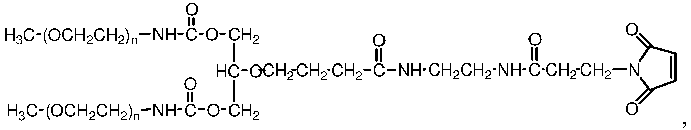 thereby forming a conjugate having the following structure: 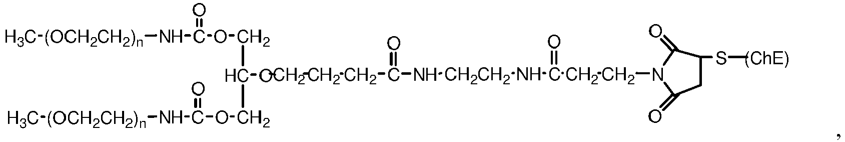 wherein:
(for each structure) each (n) is independently an integer having a value of from 2 to 4000; and
ChE is a residue of cholinesterase moiety.

An additional exemplary conjugate can be formed using a reagent: 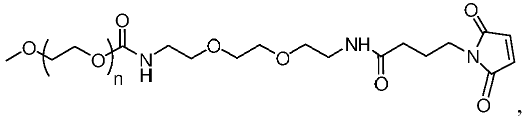 thereby forming a conjugate having the following structure: 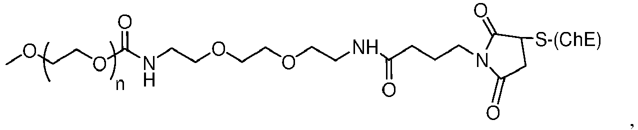 wherein:
(for each structure) (n) is independently an integer having a value of from 2 to 4000; and
ChE is a residue of cholinesterase moiety.

Conjugates can be formed using thiol-selective polymeric reagents in a number of ways. For example, the cholinesterase moiety -- optionally in a suitable buffer (including amine-containing buffers, if desired) -- is placed in an aqueous media at a pH of about 7-8 and the thiol-selective polymeric reagent is added at a molar excess. The reaction is allowed to proceed for about 0.5 to 2 hours, although reaction times of greater than 2 hours (e.g., 5 hours, 10 hours, 12 hours, and 24 hours) can be useful if PEGylation yields are determined to be relatively low. Polymeric reagents that can be used in this approach are polymeric reagents bearing a reactive group selected from the group consisting of maleimide, sulfone (e.g., vinyl sulfone), and thiol (e.g., functionalized thiols such as an ortho pyridinyl or "OPSS").

As indicated previously, a thiol-selective polymeric reagent (e.g., a polymeric reagent bearing a maleimide functional group) can be used to form a conjugate with a cholinesterase moiety. Under conjugation conditions, the thiol-selective polymeric reagent is reacted with a dimer form of the cholinesterase moiety (e.g., a dimer form of recombinant human BChE). The position in the cholinesterase moiety corresponding to Cys66 is selectively conjugated, and a mixture is formed wherein the mixture comprises a mono-conjugated dimer with attachment at Cys66 of one monomer subunit making up the dimer and a di-conjugated dimer with attachment at Cys66 of each of the two monomer subunits making up the dimer (e.g., a mixture comprising monoPEGylated dimers with attachment at Cys66 of one subunit making up the dimer and diPEGylated dimers with attachment at Cys66 for each of the two subunits making up the dimer).

A reducing step is carried out in a method for preparing conjugates. A reducing step can be carried out using techniques known to one of ordinary skill in the art. For example, a reducing step can be carried out by subjecting a protein to reducing conditions, e.g., addition of a reducing agent such as 2-mercaptoethanol, dithiothreitol, or tris(2-carboxyethyl)phosphine.

In accordance with the claims, the reducing step is carried out following an initial conjugation reaction (with, for example, a subsequent purification and/or a subsequent conjugation).

A reducing step is carried out with the mixture described above, i.e., a mixture comprising a mono-conjugated dimer with attachment at Cys66 of one monomer subunit making up the dimer and a di-conjugated dimer with attachment Cys66 of each of the two monomer subunits making up the dimer (e.g., a mixture comprising monoPEGylated dimers with attachment at Cys66 of one subunit making up the dimer and diPEGylated dimers with attachment at Cys66 for each of the two subunits making up the dimer). The result of reducing the aforementioned mixture is a reduced mixture comprising unconjugated monomer and mono-conjugated monomer. Thereafter, the reduced mixture can be purified using art-known techniques (such as ion-exchange chromatography) to substantially separate unconjugated monomer and mono-conjugated monomer to form a composition comprising substantially unconjugated monomer and a composition comprising mono-conjugated monomer. Thereafter, the reducing conditions are removed (e.g., remove or separate the reducing agent, by, for example, utilizing ion-exchange chromatography, size-exclusion chromatography, or diafiltration) from the composition comprising substantially mono-conjugated monomer with the result that disulfide bonds regenerate to form, for example, a composition comprising diconjugated dimer (e.g., diPEGylated dimer). Optionally, a tangential flow filtration ("TFF") step can performed following the removal of the reducing conditions to concentrate the composition comprising monoconjugated monomer, with the benefit of increasing the formation rate of diconjugated dimer. The aforementioned approach has the benefits of relatively high yields, e.g., above 50% of diconjugated dimer (e.g., diPEGylated dimer), simplified product characterization and relatively reduced need for polymeric reagent.

With respect to polymeric reagents, those described here and elsewhere can be purchased from commercial sources or prepared from commercially available starting materials. In addition, methods for preparing the polymeric reagents are described in the literature.

The attachment between the cholinesterase moiety and the non-peptidic water-soluble polymer can be direct, wherein no intervening atoms are located between the cholinesterase moiety and the polymer, or indirect, wherein one or more atoms are located between the cholinesterase moiety and the polymer. With respect to the indirect attachment, a "spacer moiety" serves as a linker between the residue of the cholinesterase moiety and the water-soluble polymer. The one or more atoms making up the spacer moiety can include one or more of carbon atoms, nitrogen atoms, sulfur atoms, oxygen atoms, and combinations thereof. The spacer moiety can comprise an amide, secondary amine, carbamate, thioether, and/or disulfide group. Non-limiting examples of specific spacer moieties include those selected from the group consisting of -O-, -S-, -S-S-, -C(O)-, -C(O)-NH-, -NH-C(O)-NH-, -O-C(O)-NH-, -C(S)-, -CH₂-, -CH₂-CH₂-, -CH₂-CH₂-CH₂-, -CH₂-CH₂-CH₂-CH₂-, -O-CH₂-, -CH₂-O-, -O-CH₂-CH₂-, -CH₂-O-CH₂-, -CH₂-CH₂-O-, -O-CH₂-CH₂-CH₂-, -CH₂-O-CH₂-CH₂-, -CH₂-CH₂-O-CH₂-, -CH₂-CH₂-CH₂-O-, -O-CH₂-CH₂-CH₂-CH₂-, -CH₂-O-CH₂-CH₂-CH₂-, -CH₂-CH₂-O-CH₂-CH₂-, -CH₂-CH₂-CH₂-O-CH₂-, -CH₂-CH₂-CH₂-CH₂-O-, -C(O)-NH-CH₂-, -C(O)-NH-CH₂-CH₂-, -CH₂-C(O)-NH-CH₂-, -CH₂-CH₂-C(O)-NH-, -C(O)-NH-CH₂-CH₂-CH₂-, -CH₂-C(O)-NH-CH₂-CH₂-, -CH₂-CH₂-C(O)-NH-CH₂-, -CH₂-CH₂-CH₂-C(O)-NH-, -C(O)-NH-CH₂-CH₂-CH₂-CH₂-, -CH₂-C(O)-NH-CH₂-CH₂-CH₂-, -CH₂-CH₂-C(O)-NH-CH₂-CH₂-, -CH₂-CH₂-CH₂-C(O)-NH-CH₂-, -CH₂-CH₂-CH₂-C(O)-NH-CH₂-CH₂-, -CH₂-CH₂-CH₂-CH₂-C(O)-NH-, -C(O)-O-CH₂-, -CH₂-C(O)-O-CH₂-, -CH₂-CH₂-C(O)-O-CH₂-, -C(O)-O-CH₂-CH₂-, -NH-C(O)-CH₂-, -CH₂-NH-C(O)-CH₂-, -CH₂-CH₂-NH-C(O)-CH₂-, -NH-C(O)-CH₂-CH₂-, -CH₂-NH-C(O)-CH₂-CH₂-, -CH₂-CH₂-NH-C(O)-CH₂-CH₂-, -C(O)-NH-CH₂-, -C(O)-NH-CH₂-CH₂-, -O-C(O)-NH-CH₂-, -O-C(O)-NH-CH₂-CH₂-, -NH-CH₂-, -NH-CH₂-CH₂-, -CH₂-NH-CH₂-, -CH₂-CH₂-NH-CH₂-, - C(O)-CH₂-, -C(O)-CH₂-CH₂-, -CH₂-C(O)-CH₂-, -CH₂-CH₂-C(O)-CH₂-, -CH₂-CH₂-C(O)-CH₂-CH₂-, -CH₂-CH₂-C(O)-, -CH₂-CH₂-CH₂-C(O)-NH-CH₂-CH₂-NH-, -CH₂-CH₂-CH₂-C(O)-NH-CH₂-CH₂-NH-C(O)-, -CH₂-CH₂-CH₂-C(O)-NH-CH₂-CH₂-NH-C(O)-CH₂-, -CH₂-CH₂-CH₂-C(O)-NH-CH₂-CH₂-NH-C(O)-CH₂-CH₂-, -O-C(O)-NH-[CH₂]ₕ-(OCH₂CH₂)ⱼ-, bivalent cycloalkyl group, -O-, -S-, an amino acid, -N(R⁶)-, and combinations of two or more of any of the foregoing, wherein R⁶ is H or an organic radical selected from the group consisting of alkyl, substituted alkyl, alkenyl, substituted alkenyl, alkynyl, substituted alkynyl, aryl and substituted aryl, (h) is zero to six, and (j) is zero to 20. Other specific spacer moieties have the following structures: -C(O)-NH-(CH₂)₁₋₆-NH-C(O)-, -NH-C(O)-NH-(CH₂)₁₋₆-NH-C(O)-, and -O-C(O)-NH-(CH₂)₁₋₆-NH-C(O)-, wherein the subscript values following each methylene indicate the number of methylenes contained in the structure, e.g., (CH₂)₁₋₆ means that the structure can contain 1, 2, 3, 4, 5 or 6 methylenes. Additionally, any of the above spacer moieties may further include an ethylene oxide oligomer chain comprising 1 to 20 ethylene oxide monomer units [i.e., - (CH₂CH₂O)₁₋₂₀]. That is, the ethylene oxide oligomer chain can occur before or after the spacer moiety, and optionally in between any two atoms of a spacer moiety comprised of two or more atoms. Also, the oligomer chain would not be considered part of the spacer moiety if the oligomer is adjacent to a polymer segment and merely represent an extension of the polymer segment.

### Compositions

The conjugates are typically part of a composition. Generally, the composition comprises a plurality of conjugates, preferably although not necessarily, each conjugate is comprised of the same cholinesterase moiety (i.e., within the entire composition, only one type of cholinesterase moiety is found). In addition, the composition can comprise a plurality of conjugates wherein any given conjugate is comprised of a moiety selected from the group consisting of two or more different cholinesterase moieties (i.e., within the entire composition, two or more different cholinesterase moieties are found). Optimally, however, substantially all conjugates in the composition (e.g., 85% or more of the plurality of conjugates in the composition) are each comprised of the same cholinesterase moiety.

The composition of the invention can comprise a single conjugate species or a mixture of conjugate species. The compositions can also comprise other conjugates having four, five, six, seven, eight or more polymers attached to any given moiety having cholinesterase activity. In addition, the invention includes instances wherein the composition comprises a plurality of conjugates according to the invention, each conjugate comprising two water-soluble polymers covalently attached to one butyrylcholinesterase dimer moiety.

With respect to the conjugates in the composition, the composition will satisfy one or more of the following characteristics: at least about 85% of the conjugates in the composition will have two polymers attached to the butyrylcholinesterase moiety; at least about 95% of the conjugates in the composition will have two polymers attached to the butyrylcholinesterase moiety; at least about 99% of the conjugates in the composition will have two polymers attached to the cholinesterase moiety. It is understood that a reference to a range of polymers, e.g., "from x to y polymers," contemplates a number of polymers x to y *inclusive* (that is, for example, "from one to three polymers" contemplates one polymer, two polymers and three polymers, "from one to two polymers" contemplates one polymer and two polymers, and so forth).

In one or more embodiments, it is preferred that the conjugate-containing composition is free or substantially free of albumin. It is also preferred that the composition is free or substantially free of proteins that do not have cholinesterase activity. Thus, it is preferred that the composition is 85%, more preferably 95%, and most preferably 99% free of albumin. Additionally, it is preferred that the composition is 85%, more preferably 95%, and most preferably 99% free of any protein that does not have cholinesterase activity. To the extent that albumin is present in the composition, exemplary compositions of the invention are substantially free of conjugates comprising a poly(ethylene glycol) polymer linking a residue of a cholinesterase moiety to albumin.

Control of the desired number of polymers for any given moiety can be achieved by selecting the proper polymeric reagent, the ratio of polymeric reagent to the cholinesterase moiety, temperature, pH conditions, and other aspects of the conjugation reaction. In addition, reduction or elimination of the undesired conjugates (e.g., those conjugates having four or more attached polymers) can be achieved through purification means.

For example, the polymer-cholinesterase moiety conjugates can be purified to obtain/isolate different conjugated species. Specifically, the product mixture can be purified to obtain an average of anywhere from one, two, three, four, five or more PEGs per cholinesterase moiety, typically one, two or three PEGs per cholinesterase moiety. The strategy for purification of the final conjugate reaction mixture will depend upon a number of factors, including, for example, the molecular weight of the polymeric reagent employed, the particular cholinesterase moiety, the desired dosing regimen, and the residual activity and *in vivo* properties of the individual conjugate(s).

If desired, conjugates having different molecular weights can be isolated using gel filtration chromatography and/or ion exchange chromatography. That is to say, gel filtration chromatography is used to fractionate differently numbered polymer-to-cholinesterase moiety ratios (e.g., 1-mer, 2-mer, 3-mer, and so forth, wherein "1-mer" indicates 1 polymer to cholinesterase moiety, "2-mer" indicates two polymers to cholinesterase moiety, and so on) on the basis of their differing molecular weights (where the difference corresponds essentially to the average molecular weight of the water-soluble polymer portion). For example, in an exemplary reaction where a 35,000 Dalton protein is randomly conjugated to a polymeric reagent having a molecular weight of about 20,000 Daltons, the resulting reaction mixture may contain unmodified protein (having a molecular weight of about 35,000 Daltons), monoPEGylated protein (having a molecular weight of about 55,000 Daltons), diPEGylated protein (having a molecular weight of about 75,000 Daltons), and so forth.

While this approach can be used to separate PEG and other polymer-cholinesterase moiety conjugates having different molecular weights, this approach is generally ineffective for separating positional isoforms having different polymer attachment sites within the cholinesterase moiety. For example, gel filtration chromatography can be used to separate from each other mixtures of PEG 1-mers, 2-mers, 3-mers, and so forth, although each of the recovered conjugate compositions may contain PEG(s) attached to different reactive groups (e.g., lysine residues) within the cholinesterase moiety.

Gel filtration columns suitable for carrying out this type of separation include Superdex™ and Sephadex™ columns available from Amersham Biosciences (Piscataway, NJ). Selection of a particular column will depend upon the desired fractionation range desired. Elution is generally carried out using a suitable buffer, such as phosphate or acetate. The collected fractions may be analyzed by a number of different methods, for example, (i) absorbance at 280 nm for protein content, (ii) dye-based protein analysis using bovine serum albumin (BSA) as a standard, (iii) iodine testing for PEG content (Sims et al. (1980) Anal. Biochem, 107:60-63), (iv) sodium dodecyl sulfate polyacrylamide gel electrophoresis (SDS PAGE), followed by staining with barium iodide, and (v) high performance liquid chromatography (HPLC).

Separation of positional isoforms is carried out by reverse phase chromatography using a reverse phase-high performance liquid chromatography (RP-HPLC) using a suitable column (e.g., a C18 column or C3 column, available commercially from companies such as Amersham Biosciences or Vydac) or by ion exchange chromatography using an ion exchange column, e.g., a Sepharose™ ion exchange column available from Amersham Biosciences. Either approach can be used to separate polymer-active agent isomers having the same molecular weight (i.e., positional isoforms).

The compositions are preferably substantially free of proteins that do not have cholinesterase activity. In addition, the compositions preferably are substantially free of all other noncovalently attached water-soluble polymers. In some circumstances, however, the composition can contain a mixture of polymer- cholinesterase moiety conjugates and unconjugated cholinesterase moiety.

The composition of the invention further comprises a pharmaceutically acceptable excipient. If desired, the pharmaceutically acceptable excipient can be added to a conjugate to form a composition.

Exemplary excipients include, without limitation, those selected from the group consisting of carbohydrates, inorganic salts, antimicrobial agents, antioxidants, surfactants, buffers, acids, bases, and combinations thereof.

A carbohydrate such as a sugar, a derivatized sugar such as an alditol, aldonic acid, an esterified sugar, and/or a sugar polymer may be present as an excipient. Specific carbohydrate excipients include, for example: monosaccharides, such as fructose, maltose, galactose, glucose, D-mannose and sorbose; disaccharides, such as lactose, sucrose, trehalose and cellobiose; polysaccharides, such as raffinose, melezitose, maltodextrins, dextrans and starches; and alditols, such as mannitol, xylitol, maltitol, lactitol, xylitol, sorbitol (glucitol), pyranosyl sorbitol and myoinositol.

The excipient can also include an inorganic salt or buffer such as citric acid, sodium chloride, potassium chloride, sodium sulfate, potassium nitrate, sodium phosphate monobasic, sodium phosphate dibasic, and combinations thereof.

The composition can also include an antimicrobial agent for preventing or deterring microbial growth. Nonlimiting examples of antimicrobial agents suitable for one or more embodiments of the present invention include benzalkonium chloride, benzethonium chloride, benzyl alcohol, cetylpyridinium chloride, chlorobutanol, phenol, phenylethyl alcohol, phenylmercuric nitrate, thimersol, and combinations thereof.

An antioxidant can be present in the composition as well. Antioxidants are used to prevent oxidation, thereby preventing the deterioration of the conjugate or other components of the preparation. Suitable antioxidants for use in one or more embodiments of the present invention include, for example, ascorbyl palmitate, butylated hydroxyanisole, butylated hydroxytoluene, hypophosphorous acid, monothioglycerol, propyl gallate, sodium bisulfite, sodium formaldehyde sulfoxylate, sodium metabisulfite, and combinations thereof.

A surfactant can be present as an excipient. Exemplary surfactants include: polysorbates, such as "Tween 20" and "Tween 80," and pluronics such as F68 and F88 (both of which are available from BASF, Mount Olive, New Jersey); sorbitan esters; lipids, such as phospholipids such as lecithin and other phosphatidylcholines, phosphatidylethanolamines (although preferably not in liposomal form), fatty acids and fatty esters; steroids, such as cholesterol; and chelating agents, such as EDTA, zinc and other such suitable cations.

Acids or bases can be present as an excipient in the composition. Non-limiting examples of acids that can be used include those acids selected from the group consisting of hydrochloric acid, acetic acid, phosphoric acid, citric acid, malic acid, lactic acid, formic acid, trichloroacetic acid, nitric acid, perchloric acid, phosphoric acid, sulfuric acid, fumaric acid, and combinations thereof. Examples of suitable bases include, without limitation, bases selected from the group consisting of sodium hydroxide, sodium acetate, ammonium hydroxide, potassium hydroxide, ammonium acetate, potassium acetate, sodium phosphate, potassium phosphate, sodium citrate, sodium formate, sodium sulfate, potassium sulfate, potassium fumerate, and combinations thereof.

The amount of the conjugate (i.e., the conjugate formed between the active agent and the polymeric reagent) in the composition will vary depending on a number of factors, but will optimally be a therapeutically effective dose when the composition is stored in a unit dose container (e.g., a vial). In addition, the pharmaceutical preparation can be housed in a syringe. A therapeutically effective dose can be determined experimentally by repeated administration of increasing amounts of the conjugate in order to determine which amount produces a clinically desired endpoint.

The amount of any individual excipient in the composition will vary depending on the activity of the excipient and particular needs of the composition. Typically, the optimal amount of any individual excipient is determined through routine experimentation, i.e., by preparing compositions containing varying amounts of the excipient (ranging from low to high), examining the stability and other parameters, and then determining the range at which optimal performance is attained with no significant adverse effects.

Generally, however, the excipient will be present in the composition in an amount of about 1% to about 99% by weight, preferably from about 5% to about 98% by weight, more preferably from about 15 to about 95% by weight of the excipient, with concentrations less than 30% by weight most preferred.

These foregoing pharmaceutical excipients along with other excipients are described in "Remington: The Science & Practice of Pharmacy", 19th ed., Williams & Williams, (1995), the "Physician's Desk Reference", 52nd ed., Medical Economics, Montvale, NJ (1998), and Kibbe, A.H., Handbook of Pharmaceutical Excipients, 3rd Edition, American Pharmaceutical Association, Washington, D.C., 2000.

The compositions encompass all types of formulations and in particular those that are suited for injection, e.g., powders or lyophilates that can be reconstituted as well as liquids. Examples of suitable diluents for reconstituting solid compositions prior to injection include bacteriostatic water for injection, dextrose 5% in water, phosphate-buffered saline, Ringer's solution, saline, sterile water, deionized water, and combinations thereof. With respect to liquid pharmaceutical compositions, solutions and suspensions are envisioned.

The compositions of one or more embodiments of the present invention are typically, although not necessarily, administered via injection and are therefore generally liquid solutions or suspensions immediately prior to administration. The pharmaceutical preparation can also take other forms such as syrups, creams, ointments, tablets and powders. Other modes of administration are also included, such as pulmonary, rectal, transdermal, transmucosal, oral, intrathecal, subcutaneous and intra-arterial.

There is described a method for administering a conjugate as provided herein to a patient suffering from a condition that is responsive to treatment with conjugate. The method comprises administering to a patient, generally via injection, a therapeutically effective amount of the conjugate (preferably provided as part of a pharmaceutical composition). As previously described, the conjugates can be injected (e.g., intramuscularly, subcutaneously and parenterally). Suitable formulation types for parenteral administration include ready-for-injection solutions, dry powders for combination with a solvent prior to use, suspensions ready for injection, dry insoluble compositions for combination with a vehicle prior to use, and emulsions and liquid concentrates for dilution prior to administration.

The method of administering may be used to treat any condition that can be remedied or prevented by administration of the conjugate. Those of ordinary skill in the art appreciate which conditions a specific conjugate can effectively treat. For example, the conjugates can be used either alone or in combination with other pharmacotherapy to treat patients suffering from exposure to organophosphates. Advantageously, the conjugate can be administered to the patient prior to, simultaneously with, or after administration of another active agent.

The actual dose to be administered will vary depending upon the age, weight, and general condition of the subject as well as the severity of the condition being treated, the judgment of the health care professional, and conjugate being administered. Therapeutically effective amounts are known to those skilled in the art and/or are described in the pertinent reference texts and literature. Generally, a therapeutically effective amount will range from about 0.001 mg to 100 mg, preferably in doses from 0.01 mg/day to 75 mg/day, and more preferably in doses from 0.10 mg/day to 50 mg/day. A given dose can be periodically administered up until, for example, symptoms of organophosphate poisoning lessen and/or are eliminated entirely.

The unit dosage of any given conjugate (again, preferably provided as part of a pharmaceutical preparation) can be administered in a variety of dosing schedules depending on the judgment of the clinician, needs of the patient, and so forth. The specific dosing schedule will be known by those of ordinary skill in the art or can be determined experimentally using routine methods. Exemplary dosing schedules include, without limitation, administration once daily, three times weekly, twice weekly, once weekly, twice monthly, once monthly, and any combination thereof. Once the clinical endpoint has been achieved, dosing of the composition is halted.

One advantage of administering certain conjugates described herein is that individual water-soluble polymer portions can be cleaved when a hydrolytically degradable linkage is included between the residue of cholinesterase moiety and water-soluble polymer. Such a result is advantageous when clearance from the body is potentially a problem because of the polymer size. Optimally, cleavage of each water-soluble polymer portion is facilitated through the use of physiologically cleavable and/or enzymatically degradable linkages such as amide, carbonate or ester-containing linkages. In this way, clearance of the conjugate (via cleavage of individual water-soluble polymer portions) can be modulated by selecting the polymer molecular size and the type functional group that would provide the desired clearance properties. One of ordinary skill in the art can determine the proper molecular size of the polymer as well as the cleavable functional group. For example, one of ordinary skill in the art, using routine experimentation, can determine a proper molecular size and cleavable functional group by first preparing a variety of polymer derivatives with different polymer weights and cleavable functional groups, and then obtaining the clearance profile (e.g., through periodic blood or urine sampling) by administering the polymer derivative to a patient and taking periodic blood and/or urine sampling. Once a series of clearance profiles have been obtained for each tested conjugate, a suitable conjugate can be identified.

### EXPERIMENTAL

The practice of the invention will employ, unless otherwise indicated, conventional techniques of organic synthesis, biochemistry and protein purification, which are within the skill of the art. Such techniques are fully explained in the literature. See, for example, J. March, Advanced Organic Chemistry: Reactions Mechanisms and Structure, 4th Ed. (New York: Wiley-Interscience, 1992), *supra.*

In the following prophetic examples, efforts have been made to ensure accuracy with respect to numbers used (e.g., amounts, temperatures, etc.) but some experimental error and deviation should be taken into account. Unless indicated otherwise, temperature is in degrees C and pressure is at or near atmospheric pressure at sea level. Each of the following examples is considered to be instructive to one of ordinary skill in the art for carrying out one or more of the embodiments described herein.

An aqueous solution ("stock solution") comprising the cholinesterase moiety corresponding to the amino acid sequence of SEQ ID NO: 2, the mature protein sequence, was obtained for use in the examples. The concentration of the stock solution varied between 1 and 100 mg/mL.

### SDS-PAGE analysis

Samples were analyzed by sodium dodecyl sulfate-polyacrylamide gel electrophoresis (SDS-PAGE) using the Invitrogen NuPAGE system and Novex 3-8% Tris-acetate pre-cast gels (Invitrogen, Carlsbad, CA). Samples were prepared, loaded on the gel and electrophoresis performed as described by the manufacturer.

### Anion Exchange chromatography

A Q-FF Sepharose (GE Healthcare) anion exchange column with a bed volume of approximately 100 ml was prepared using standard methods. The column was connected to a GE Healthcare (Chalfont St. Giles, UK) AKTA basic or higher level system to purify the prepared PEG-rChE conjugates. Details for the purification process are described below.

### RP-HPLC Analysis

Reversed-phase chromatography (RP-HPLC) analysis was performed on an Agilent (Santa Clara, CA) 1100 HPLC system. Samples were analyzed using a Agilent Zorbax 300SB-C8 (P/N 863973-906, 4.6 X 150 mm, 3.5 µm particle size, 300 Å pore size) column. The flow rate of the column was 0.5 ml/min. The mobile phases were 0.1% TFA in water (solvent A) and 0.1% TFA in acetonitrile (solvent B).

### Examples 1A - 1D

### Conjugation Via the Cysteine Side Chain

As previously stated, conjugation of a cholinesterase moiety via a thiol-containing cysteine side chain ideally preserves existing disulfide bonds. With regard to the mature form of butyrylcholinesterase, there exists a single cysteine residue (Cys66) that is a non-disulfide bond-affected cysteines. Lockridge et al. (1987) J. Biol. Chem. 262(27): 12945-12952. Lockridge et al. also report that this cysteine was not modifiable via alkylation, presumably because this cysteine is "buried" within the secondary and tertiary structure of the protein. Thus, the ability to conjugate at this location would be unexpected.

Examples 1A through 1D were carried out using the general approach outlined below.

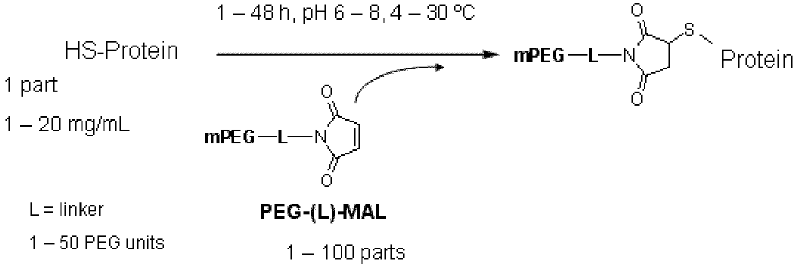

The recombinant form of the protein exists as a homo-dimer with the two identical subunits linked via a single disulfide bond between Cys571 in each subunit. The method describes the conditions to achieve a relatively high level of PEGylation by addition of one reagent per monomer protein unit under conditions where the protein is maintained as a dimer. In this way, each identical subunit is PEGylated substantially at the Cys66 position and not the Cys571 position.

### Example 1A

### PEGylation of rBChE with a Linear 20kDa PEG Bearing a Maleimide Group

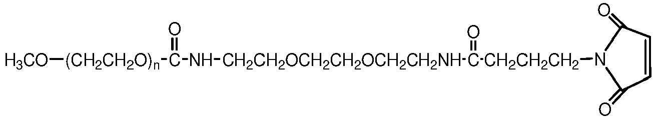

20kDa PEG Bearing a Maleimide Group
(n defined to provide a ∼20kDa PEG)

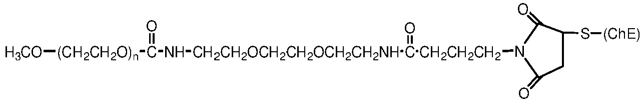

### Example 1A Conjugate

### (each n defined to provide a ∼20kDa PEG)

The starting concentration of the butyrylcholinesterase protein stock solution was ±90 mg/mL and the protein was dissolved in a buffer containing 10 mM NaPO₄ (pH 7.4), 1 mM EDTA and 35 mM NaCl. One gram of protein (11 mL of the above stock solution) was diluted with 8.1 mL of dilution buffer (2 mM NaPO₄, 1 mM EDTA (pH 7.4)) such that the final protein concentration was 52-53 mg/mL.

While stirring this protein solution, 0.74 mL of Tris buffer (1M Tris, pH 8.2) was added. This was followed by addition of 0.238 mL of Tris base (1 M Tris base). The resulting pH of the solution was pH 8.30.

In a separate container an amount of the PEG reagent, equal to 6 mol equivalents of the protein quantity, was dissolved in PEG dilution buffer (2 mM NaPO₄, 1 mM EDTA, pH 6.1) to a 16.7% (w/v) solution. While stirring the protein solution the PEG reagent solution was added to the diluted protein solution. This mixture is hereafter referred to as the PEGylation reaction. The PEGylation reaction was allowed to stir for six hours at room temperature (22 °C).

After this time a second PEG reagent solution was made by dissolving an amount of PEG reagent equal to 4 mol equivalents of the protein quantity in PEG dilution buffer. While continuing to stir the PEGylation reaction this additional PEG reagent solution was added to the PEGylation reaction. The PEGylation reaction was allowed to stir for an additional 6-18 hours at room temperature (22 °C). The PEGylation reaction was then stored at 4 °C until the PEGylation products were purified, but usually within 48 hours.

### Example 1B

### PEGylation of rBChE with a Linear 30kDa PEG Bearing a Maleimide Group

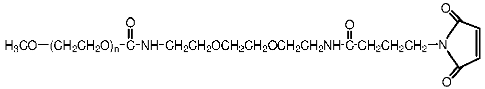

30kDa PEG Bearing a Maleimide Group
(n defined to provide a ∼30kDa PEG)

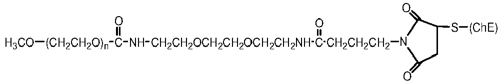

### Example 1B Conjugate

### (n defined to provide a ∼30kDa PEG)

The starting concentration of the butyrylcholinesterase protein stock solution was ±90 mg/mL and the protein was dissolved in a buffer containing 10 mM NaPO₄ (pH 7.4), 1 mM EDTA and 35 mM NaCl. One gram of protein (11 mL of the above stock solution) was diluted with 8.1 mL of dilution buffer (2 mM NaPO₄, 1 mM EDTA (pH 7.4)) such that the final protein concentration was 52-53 mg/mL.

While stirring this protein solution, 0.74 mL of Tris buffer (1M Tris, pH 8.2) was added. This was followed by addition of 0.238 mL of Tris base (1 M Tris base). The resulting pH of the solution was pH 8.30.

In a separate container an amount of the PEG reagent, equal to 6 mol equivalents of the protein quantity, was dissolved in PEG dilution buffer (2 mM NaPO₄, 1 mM EDTA, pH 6.1) to a 16.7% (w/v) solution. While stirring the protein solution the PEG reagent solution was added to the diluted protein solution. This mixture is hereafter referred to as the PEGylation reaction. The PEGylation reaction was allowed to stir for six hours at room temperature (22 °C).

After this time a second PEG reagent solution was made by dissolving an amount of PEG reagent equal to 4 mol equivalents of the protein quantity in PEG dilution buffer. While continuing to stir the PEGylation reaction this additional PEG reagent solution was added to the PEGylation reaction. The PEGylation reaction was allowed to stir for an additional 6-18 hours at room temperature (22 °C). The PEGylation reaction was then stored at 4 °C until the PEGylation products were purified, but usually within 48 hours.

### Example 1C

### PEGylation of rBChE with a Branched 40kDa PEG Bearing a Maleimide Group

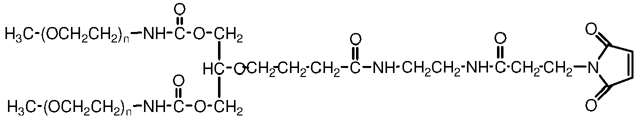

Branched 40kDa PEG Bearing a Maleimide Group
(each n defined to provide a ∼20kDa PEG)

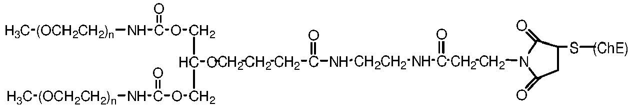

### Example 1C Conjugate

### (each n defined to provide a ∼20kDa PEG)

The starting concentration of the butyrylcholinesterase protein stock solution was ±90 mg/mL and the protein was dissolved in a buffer containing 10 mM NaPO₄ (pH 7.4), 1 mM EDTA and 35 mM NaCl. One gram of protein (11 mL of the above solution) was diluted with 8.1 mL of dilution buffer (2 mM NaPO₄, 1 mM EDTA (pH 7.4)) such that the final protein concentration was 52-53 mg/mL.

While stirring this protein solution, 0.74 mL of Tris buffer (1M Tris, pH 8.2) was added. This was followed by addition of 0.238 mL of Tris base (1 M Tris base). The resulting pH of the solution was pH 8.30.

In a separate container an amount of the PEG reagent, equal to 6 mol equivalents of the protein quantity, was dissolved in PEG dilution buffer (2 mM NaPO₄, 1 mM EDTA, pH 6.1) to a 16.7% (w/v) solution. While stirring the protein solution the PEG reagent solution was added to the diluted protein solution. This mixture is hereafter referred to as the PEGylation reaction. The PEGylation reaction was allowed to stir for 6 hours at room temperature (22 °C).

After this time a second PEG reagent solution was made by dissolving an amount of PEG reagent equal to 4 mol equivalents of the protein quantity in PEG dilution buffer. While continuing to stir the PEGylation reaction this additional PEG reagent solution was added to the PEGylation reaction. The PEGylation reaction was allowed to stir for an additional 6-18 hours at room temperature (22 °C). The PEGylation reaction was then stored at 4 °C until the PEGylation products were purified, but usually within 48 hours.

### Example ID

### PEGylation of rBChE with a Branched 60kDa PEG Bearing a Maleimide Group

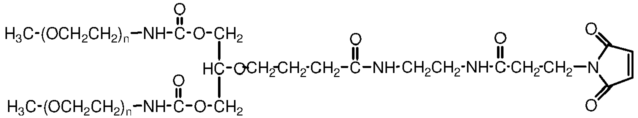

### Branched 60kDa PEG Bearing a Maleimide Group (each n defined to provide a ∼30kDa PEG)

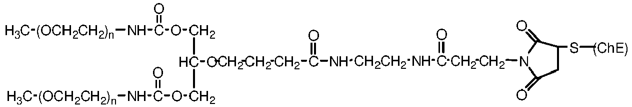

### Example 1D Conjugate

### (each n defined to provide a ∼30kDa PEG)

The starting concentration of the butyrylcholinesterase protein stock solution was ±90 mg/mL and the protein was dissolved in a buffer containing 10 mM NaPO₄ (pH 7.4), 1 mM EDTA and 35 mM NaCl. One gram of protein (11 mL of the above stock solution) was diluted with 8.1 mL of dilution buffer (2 mM NaPO₄, 1 mM EDTA (pH 7.4)) such that the final protein concentration was 52-53 mg/mL.

While stirring this protein solution, 0.74 mL of Tris buffer (1M Tris, pH 8.2) was added. This was followed by addition of 0.238 mL of Tris base (1 M Tris base). The resulting pH of the solution was pH 8.30.

In a separate container an amount of the PEG reagent, equal to 6 mol equivalents of the protein quantity, was dissolved in PEG dilution buffer (2 mM NaPO₄, 1 mM EDTA, pH 6.1) to a 16.7% (w/v) solution. While stirring the protein solution the PEG reagent solution was added to the diluted protein solution. This mixture is hereafter referred to as the PEGylation reaction. The PEGylation reaction was allowed to stir for six hours at room temperature (22 °C).

After this time a second PEG reagent solution was made by dissolving an amount of PEG reagent equal to 4 mol equivalents of the protein quantity in PEG dilution buffer. While continuing to stir the PEGylation reaction this additional PEG reagent solution was added to the PEGylation reaction. The PEGylation reaction was allowed to stir for an additional 6-18 hours at room temperature (22 °C). The PEGylation reaction was then stored at 4 °C until the PEGylation products were purified, but usually within 48 hours.

### Example 2

### Alternative PEGvlation Conditions Achieving 65-70% PEGvlation Yield Using mPEG-40k-Maleimide

The reaction time for this PEGylation reaction was six days at 10 °C with stirring using a magnetic stir bar and plate.

PEG reagent was added to a stock solution in batch mode with stirring, adding 1 mol equivalent of dry PEG reagent on each day as shown in the Table 4, below.

To achieve a high level of PEGylation (i.e., > 65% PEGylation with respect to the monomer), the concentration of the protein was maintained at the highest possible level. Under these conditions, the reaction mixture was "milky/cloudy" due to the formation of a reversible protein aggregate. However, if more than one mol equivalent of PEG was added without minimal dilution of the PEGylation reaction, aggregation was too great (determined empirically) and the PEGylation efficiency was reduced. Therefore, before the addition of dry PEG, the PEGylation reaction was diluted with buffer as shown in the Table 4, below.

For the PEG reagent additions where reaction dilution buffer is also added, the PEG reagent could also be dissolved in the buffer before adding the mixture to the PEGylation reaction.

The protein was dissolved in buffer containing 10 mM NaPO₄ (pH 7.3), 1 mM EDTA and 35 mM NaCl (reaction dilution buffer) at a starting concentration of 83 mg/mL and the reaction quantities below describe PEGylation of 20 grams of rhBChE protein in a starting volume of 241 mL.

Buffer and PEG reagent additions were made at room temperature according to the specifications set forth in Table 4.

**Table 4**

| Specification of Additions | | |
|---|---|---|
| Day | Reaction dilution buffer added (mL) | PEG reagent added (grams) |
| 0 | 0 | 10.35 |
| 1 | 160 | 10.35 |
| 2 | 160 | 10.35 |
| 3 | 160 | 10.35 |
| 4 | 160 | 10.35 |
| 5 | 0 | 10.35 |
| 6 | Reaction stopped by storing at 4 °C | - |

After this reaction the PEGylated protein was purified as described in Example 3.

### Example 3

### Purification of Di-PEGvlated Dimer

The PEGylation method described in Examples 1A-1D and 2 generates a protein solution where 65 to 70% of the protein is PEGylated with respect to the monomer form of the protein. The PEGylation reaction was analyzed by RP-HPLC after the protein was reduced to monomer. However, the biological form desired from this reaction was the di-PEGylated dimer and the PEGylation reaction did not produce fully PEGylated dimer. The level of PEGylation could be marginally increased by further additions of PEG reagent, but ultimately the moderate increase in PEGylation would be offset by the increased cost of the PEG reagent. Analysis of reaction mixtures showed that approximately 50% of the reaction mixture was in the form of mono-PEGylated dimer and 45% in the form of di-PEGylated dimer. Furthermore, after purification, only 30 to 35% of di-PEGylated dimer could be recovered. The level of recovery would be too low for an economically viable process.

A method is therefore described where substantially all the mono-PEGylated monomer form of the protein which was present in the mono-PEGylated dimer fraction could be recovered and converted to di-PEGylated dimer. This method enabled a total process yield of at least 55 to 60%.

For purification of the 1 gram PEGylation reaction, a 22 mm diameter anion exchange column (Q-Sepharose fast flow) was packed (using methods known to those skilled in the art) such that the bed volume would result in a 5 mg/mL protein loading. The column was equilibrated in chromatography buffer A (10 mM NaPO₄ (pH 7.8), 1 mM EDTA, 5 mM cysteine).

The protein in the PEGylation reaction was reduced to the monomer form by addition of 1 PEGylation reaction volume of reducing buffer (10 mM NaPO₄ (pH 7.8), 1 mM EDTA, 20 mM cysteine) and incubating at room temperature for one hour.

Thereafter, seven PEGylation reaction volumes of chromatography buffer A and one PEGylation reaction volume of water were added resulting in a 10-fold dilution of the original PEGylation reaction.

An appropriate chromatography instrument was programmed so that the entire diluted PEGylation reaction was loaded onto the column and any unbound proteins washed out with two column bed volumes of chromatography buffer A. Thereafter, several gradient steps were used to elute the PEGylated monomer of BChE.

Gradient step 1 was a continuous gradient from 0 to 25 % chromatography buffer B (equivalent to buffer A but also containing 0.5 M NaCl) over 2.5 bed volumes. Fractions were collected.

Gradient step 2 was a hold step at 25 % buffer B for 2.5 bed volumes. Fractions were collected.

Gradient step 3 was a direct step to 100% B followed by a hold step at 100% B (gradient step 4) for 2 bed volumes. Fractions were collected.

The mono-PEGylated monomer eluted over a broad peak during gradient steps 1 and 2. The non-PEGylated monomer eluted during gradient step 4.

The column was regenerated using standard methods.

Appropriate fractions were pooled and buffer exchanged / concentrated using Tangential Flow Filtration (TFF) and standard methods as described by the manufacturer of the filtration units.

The mono-PEGylated monomer solution was concentrated by TFF to a protein concentration of 25 mg/mL and seven buffer volume changes of TFF buffer (10 mM NaPO₄ (pH 7.5), 1 mM EDTA, 35 mM NaCl) were applied. The solution was filter sterilized using a 0.22 µm filtration unit.

At this point, the cysteine used to reduce the protein to monomer had been removed (by the TFF process) from the protein solution and incubation at room temperature for 48 hours followed by storage at 4 °C allowed the dimer form of the protein to regenerate. The final product was therefore the di-PEGylated dimer form of the protein.

### Example 4 (not according to the invention)

### PEGylation of rChE with Branched mPEG-N-Hydroxysuccinimide Derivative, 40kDa

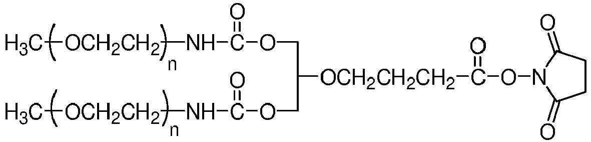

PEGylation reactions are designed such that after addition of all the reaction components and buffers, the final rChE concentration is 2.5 mg/ml. PEG2-NHS, 40kDa, stored at -20 °C under argon, is warmed to ambient temperature. A quantity of the PEG reagent equal to 10 - 50 mol equivalents of the rChE to be PEGylated is weighed out and dissolved in 20mM sodium phosphate buffer (pH 7.5) and 1 mM EDTA to form a 12% reagent solution. The 12% PEG reagent solution is quickly added to the aliquot of stock rChE solution and stirred for 3 - 18 hours at room temperature to allow for coupling of the mPEG2-NHS to rChE via an amide linkage, resulting in a conjugate solution. The conjugate solution is quenched with a lysine solution (pH 7.5) such that the final lysine molar concentration is 10 - 100 times the PEG reagent molar concentration.

mPEG2-NHS is found to provide a relatively large molecular volume of active N-hydroxysuccinimide ("NHS") ester, which selectively reacts with lysine and terminal amines.

Using this same approach, other conjugates are prepared using mPEG2-NHS having other weight average molecular weights.

Conjugates using PEG2-NHS, 40kDa, were prepared substantially in accordance with the procedure set forth in this Example wherein PEG2-NHS, 40kDa, at a mol equivalent of 10, 25 and 50 was used in three separate attempts. The SDS-PAGE analysis of the resulting conjugate solutions is provided in FIG. 1.

### Example 5 (not according to the invention)

### PEGvlation of rChE with Linear mPEG-Butyraldehyde Derivative, 30kDa

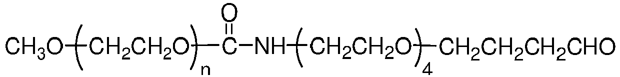

Linear mPEG-Butyraldehyde Derivative, 30kDa ("mPEG-ButyrALD")

PEGylation reactions are designed such that after addition of all the reaction components and buffers, the final rChE concentration is 2.5 mg/ml. mPEG-ButyrALD, 30kDa, stored at -20 °C under argon, is warmed to ambient temperature. A quantity of the PEG reagent equal to 10 - 50 mol equivalents of the rChE to be PEGylated is weighed out and dissolved in 20mM sodium phosphate buffer (pH 7.5) and 1 mM EDTA to form a 12% reagent solution. The 12% PEG reagent solution is added to the aliquot of stock rChE solution and stirred for 15 - 30 minutes. A reducing agent, sodium cyanoborohydride (NaCNBH₃), is then added at 10 - 100 molar excess relative to the PEG reagent and the reaction stirred for 5 - 18 hours at room temperature to ensure coupling via a secondary amine linkage to thereby form a conjugate solution.

The aldehyde group of mPEG-ButyrALD is found to react with the primary amines associated with rChE and covalently bond to them via secondary amine upon reduction by a reducing reagent such as sodium cyanoborohydride.

Using this same approach, other conjugates are prepared using mPEG-BuryrALD having other weight average molecular weights.

Conjugates using mPEG-ButyrALD, 30kDa,were prepared substantially in accordance with the procedure set forth in this Example wherein mPEG-ButyrALD, 30kDa, at a mol equivalent of 10, 25 and 50 was used in three separate attempts. The SDS-PAGE analysis of the resulting conjugate solutions is provided in FIG. 1.

### Example 6 (not according to the invention)

### PEGylation of rChE with Branched mPEG-Butyraldehyde Derivative, 40kDa

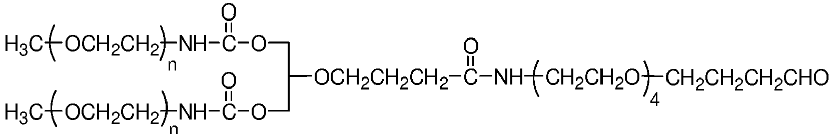

Branched mPEG-Butyraldehyde Derivative, 40kDa ("mPEG2-ButyrALD")

PEGylation reactions are designed such that after addition of all the reaction components and buffers, the final rChE concentration is 2.5 mg/ml. mPEG2-ButyrALD, 40kDa, stored at -20 °C under argon, is warmed to ambient temperature. A quantity of the PEG reagent equal to 10 - 50 mol equivalents of the rChE to be PEGylated is weighed out and dissolved in 20mM sodium phosphate buffer (pH 7.5) and 1 mM EDTA to form a 12% reagent solution. The 12% PEG reagent solution is added to the aliquot of stock rChE solution and stirred for 15 - 30 minutes. A reducing agent, sodium cyanoborohydride (NaCNBH₃), is then added at 10 - 100 molar excess relative to the PEG reagent and the reaction stirred for 5 - 18 hours at room temperature to ensure coupling via a secondary amine linkage to thereby form a conjugate solution.

The aldehyde group of mPEG2-ButyrALD is found to react with the primary amines associated with rChE and covalently bond to them via secondary amine upon reduction by a reducing reagent such as sodium cyanoborohydride.

Using this same approach, other conjugates are prepared using mPEG2-BuryrALD having other weight average molecular weights.

Conjugates using mPEG2-ButyrALD, 40kDa, were prepared substantially in accordance with the procedure set forth in this Example wherein mPEG2-ButyrALD, 40kDa, at a mol equivalent of 10, 25 and 50 was used in three separate attempts. The SDS-PAGE analysis of the resulting conjugate solutions is provided in FIG. 1.

### Example 7 (not according to the invention)

### PEGvlation of rChE with Linear mPEG-Succinimidyl α-Methylbutanoate Derivative, 30kDa

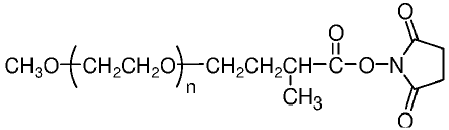

Linear mPEG-Succinimidyl α-Methylbutanoate Derivative, 30kDa ("mPEG-SMB")

PEGylation reactions are designed such that after addition of all the reaction components and buffers, the final rChE concentration is 2.5 mg/ml. mPEG-SMB, 30kDa, stored at -20 °C under argon, is warmed to ambient temperature. A quantity of the PEG reagent equal to 10 - 50 mol equivalents of the rChE to be PEGylated is weighed out and dissolved in 20mM sodium phosphate buffer (pH 7.5) and 1 mM EDTA to form a 12% reagent solution. The 12% PEG reagent solution is added to the aliquot of stock rChE solution and stirred for 5 - 18 hours at room temperature thereby resulting in a conjugate solution. The conjugate solution is quenched with a lysine solution (pH 7.5) such that the final lysine molar concentration is 10 - 100 times the PEG reagent molar concentration.

The mPEG-SMB derivative is found to provide a sterically hindered active NHS ester, which selectively reacts with lysine and terminal amines.

Using this same approach, other conjugates are prepared using mPEG-SMB having other weight average molecular weights.

### Example 8 (not according to the invention)

### PEGvlation of rChE with mPEG-PIP, 20kDa

The basic structure of the polymeric reagent is provided below:

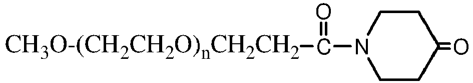

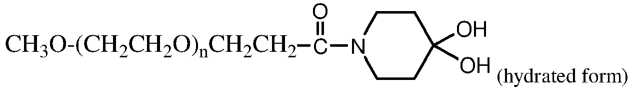

PEGylation reactions are designed such that after addition of all the reaction components and buffers, the final rChE concentration is 2.5 mg/ml. mPEG-PIP, 20kDa, stored at -20 °C under argon, is warmed to ambient temperature. A quantity of the PEG reagent equal to 10 - 50 mol equivalents of the rChE to be PEGylated is weighed out and dissolved in 20mM sodium phosphate buffer (pH 7.5) and 1 mM EDTA to form a 12% reagent solution. The 12% PEG reagent solution is added to the aliquot of stock rChE solution and stirred for 15 - 30 minutes. A reducing agent, sodium cyanoborohydride (NaCNBH₃), is then added at 10 - 100 molar excess relative to the PEG reagent and the reaction stirred for 5 - 18 hours at room temperature to ensure coupling via a secondary amine linkage (to a secondary carbon) to thereby form a conjugate solution. The conjugate solution is quenched with a lysine solution (pH 7.5) such that the final lysine molar concentration is 10 - 100 times the PEG reagent molar concentration.

The ketone group of mPEG-PIP is found to react with the primary amines associated with rChE and covalently bond to them via a secondary amine upon reduction by a reducing reagent such as sodium cyanoborohydride.

Using this same approach, other conjugates are prepared using mPEG-PIP having other weight average molecular weights.

### Example 9

### Activity of Exemplary (rChE)-PEG Conjugates

The activities of the (rChE)-PEG conjugates described in the preceding Examples are determined. All of the rChE conjugates are believed to be pharmacologically active.

## Claims

1. A di-PEGylated conjugate comprising two poly(ethylene glycol)s covalently attached to a butyrylcholinesterase dimer derived from two separate butyrylcholinesterase monomers, each butyrylcholinesterase monomer having one poly(ethylene glycol) covalently attached to cysteine residue Cys66.

2. The conjugate of claim 1, wherein the butyrylcholinesterase is recombinantly prepared.

3. The conjugate of claim 1 or claim 2, wherein each poly(ethylene glycol) is terminally capped with an end-capping moiety selected from the group consisting of hydroxy, alkoxy, substituted alkoxy, alkenoxy, substituted alkenoxy, alkynoxy, substituted alkynoxy, aryloxy and substituted aryloxy.

4. The conjugate of any one of claims 1-3, wherein each poly(ethylene glycol) has a weight-average molecular weight in a range of from about 500 Daltons to about 100,000 Daltons.

5. The conjugate of claim 1, formed by reaction of a butyrylcholinesterase with a poly(ethylene glycol) reagent bearing a maleimide group and having the structure: 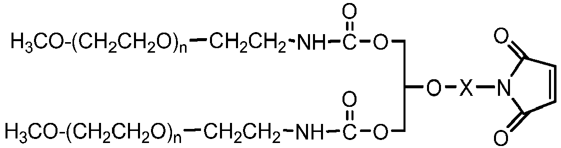 wherein:
X is a spacer moiety comprised of one or more atoms; and
each (n) is independently an integer having a value of from about 2 to about 4000.

6. The conjugate of claim 5, wherein the polymeric reagent bearing a maleimide group has the following structure: 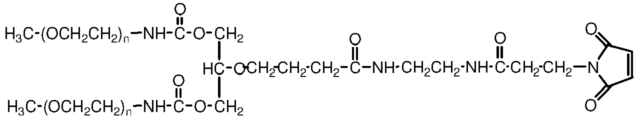 wherein each (n) is independently an integer having a value of from about 225 to about 1930.

7. The conjugate of claim 6, wherein each (n) is defined so as to provide -(OCH2CH2)- as having a molecular weight of about 20kDa.

8. The conjugate of claim 1, wherein the poly(ethylene glycol) includes the following structure: 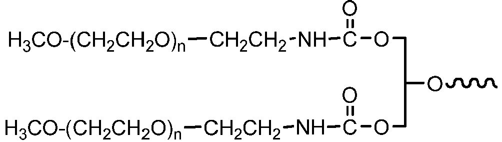 wherein each (n) is independently an integer having a value of from 2 to 4000.

9. The conjugate of claim 1, wherein each butyrylcholinesterase monomer in the dimer has the following structure: 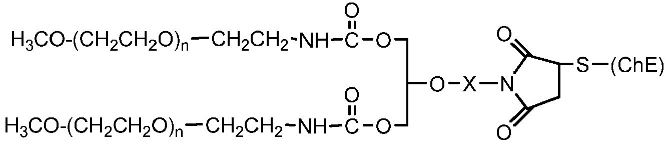 wherein:
each (n) is independently an integer having a value of from 2 to 4000;
X is a spacer moiety comprised of one or more atoms; and
ChE is a residue of a butyrylcholinesterase monomer,
or
wherein each butyrylcholinesterase monomer in the dimer has the following structure: 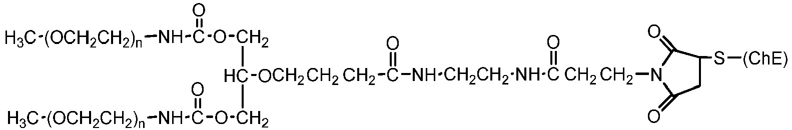 wherein each (n) is independently an integer having a value of from 2 to 4000.

10. The conjugate of any one of claims 1-4, wherein the butyrylcholinesterase moiety is glycosylated.

11. A pharmaceutical composition comprising a conjugate of any one of claims 1-10 and a pharmaceutically acceptable excipient.

12. A method for making a diconjugated butyrylcholinesterase dimer conjugate comprising:
(a) combining, under conjugation conditions, a reagent composition comprising a plurality of thiol-selective polymeric reagent molecules with a composition comprising a plurality of butyrylcholinesterase dimer molecules, to thereby form a conjugate mixture comprising monoconjugated butyrylcholinesterase dimers and diconjugated butyrylcholinesterase dimers;
(b) subjecting the conjugate mixture to reducing conditions to form a reduced mixture comprising reduced unconjugated butyrylcholinesterase monomers and reduced monoconjugated butyrylcholinesterase monomers;
(c) separating the reduced monoconjugated butyrylcholinesterase monomers from the reduced mixture to form a composition comprising reduced monoconjugated butyrylcholinesterase monomers; and
(d) removing the reducing conditions from the composition comprising reduced monoconjugated butyrylcholinesterase monomers to thereby form a composition of diconjugated butyrylcholinesterase dimers.

13. The method of claim 12, wherein the composition comprising reduced monoconjugated butyrylcholinesterase monomers is substantially free of reduced unconjugated butyrylcholinesterase monomers.

14. The method of claim 12, wherein the polymeric reagent is a poly(ethylene glycol) reagent bearing a thiol-reactive functional group;
preferably the polymeric reagent is a branched water-soluble polymer having the following structure: 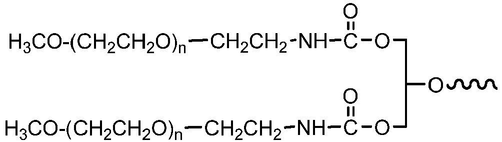 wherein each (n) is independently an integer having a value of from about 2 to 4000; or
having the following structure: 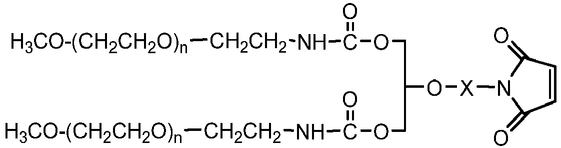 wherein:
X is a spacer moiety comprised of one or more atoms; and
each (n) is independently an integer having a value of from 2 to 4000.

15. The method of claim 14, wherein each poly(ethylene glycol) reagent has a weight-average molecular weight in the range of 5,000 to about 100,000 daltons;
preferably, each poly(ethylene glycol) reagent has a weight-average molecular weight of 20,000 daltons.

16. The method of claim 12, wherein subjecting the composition to reducing conditions comprises adding a reducing agent selected from 2-mercaptoethanol, dithiothreitol, or tris(2-carboxyethyl)phosphine to the conjugate mixture.

17. The method of claim 16, wherein removing the reducing agent comprises removing the reducing agent by ion-exchange chromatography, size exclusion chromatography, or diafiltration.

## Patentansprüche

1. Di-PEGyliertes Konjugat, umfassend zwei Poly(ethylenglykol)e, die kovalent an ein Butyrylcholinesterase-Dimer gebunden sind, das von zwei separaten Butyrylcholinesterase-Monomeren abgeleitet ist, wobei jedes Butyrylcholinesterase-Monomer ein kovalent an den Cysteinrest Cys66 gebundenes Poly(ethylenglykol) aufweist.

2. Konjugat nach Anspruch 1, bei dem die Butyrylcholinesterase rekombinant hergestellt ist.

3. Konjugat nach Anspruch 1 oder 2, bei dem jedes Poly(ethylenglycol) endständig mit einer Endverkappungseinheit verkappt ist, die ausgewählt ist aus der Gruppe bestehend aus Hydroxy, Alkoxy, substituiertem Alkoxy, Alkenoxy, substituiertem Alkenoxy, Alkynoxy, substituiertem Alkynoxy, Aryloxy und substituiertem Aryloxy.

4. Konjugat nach einem der Ansprüche 1 bis 3, bei dem jedes Poly(ethylenglykol) ein gewichtsmittleres Molekulargewicht in einem Bereich von etwa 500 Dalton bis etwa 100.000 Dalton aufweist.

5. Konjugat nach Anspruch 1, gebildet durch Reaktion einer Butyrylcholinesterase mit einem eine Maleimidgruppe tragenden Poly(ethylenglykol)-Reagenz mit der Struktur 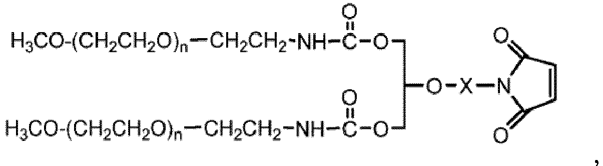 wobei:
X eine Spacer-Einheit ist, die ein oder mehrere Atome umfasst; und jedes (n) unabhängig eine ganze Zahl mit einem Wert von etwa 2 bis etwa 4000 ist.

6. Konjugat nach Anspruch 5, wobei das eine Maleimidgruppe tragende polymere Reagenz die folgende Struktur aufweist: 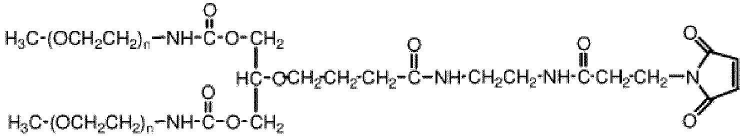 wobei jedes (n) unabhängig voneinander eine ganze Zahl mit einem Wert von etwa 225 bis etwa 1930 ist.

7. Konjugat nach Anspruch 6, wobei jedes (n) so definiert ist, dass - (OCH₂CH₂)- ein Molekulargewicht von etwa 20 kDa aufweist.

8. Konjugat nach Anspruch 1, bei dem das Poly(ethylenglykol) die folgende Struktur aufweist: 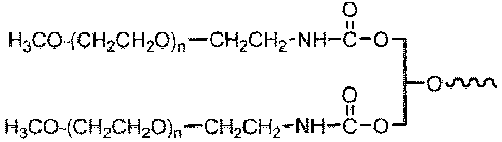 wobei jedes (n) unabhängig eine ganze Zahl mit einem Wert von 2 bis 4000 ist.

9. Konjugat nach Anspruch 1, bei dem jedes Butyrylcholinesterase-Monomer in dem Dimer die folgende Struktur aufweist: 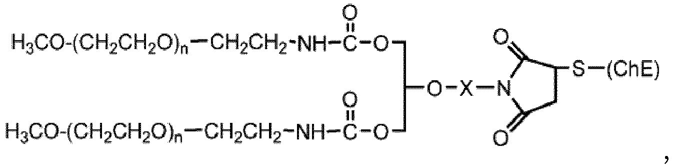 wobei:
jedes (n) unabhängig eine ganze Zahl mit einem Wert von 2 bis 4000 ist;
X eine ein oder mehrere Atome umfassende Spacer-Einheit ist; und
ChE ein Rest eines Butyrylcholinesterase-Monomers ist,
oder wobei
jedes Butyrylcholinesterase-Monomer in dem Dimer die folgende Struktur aufweist: 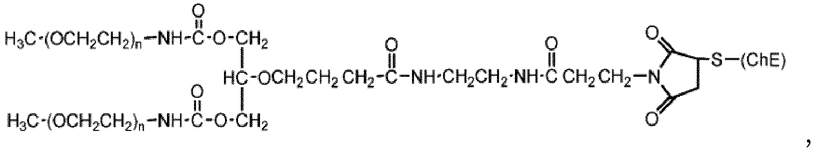 und jedes (n) unabhängig eine ganze Zahl mit einem Wert von 2 bis 4000 ist.

10. Konjugat nach einem der Ansprüche 1 bis 4, bei dem die Butyrylcholinesterase-Einheit glykosyliert ist.

11. Pharmazeutische Zusammensetzung, umfassend ein Konjugat nach einem der Ansprüche 1-10 und einen pharmazeutisch verträglichen Exzipienten.

12. Verfahren zur Herstellung eines dikonjugierten Butyrylcholinesterase-Dimer-Konjugats, umfassend:
(a) Kombinieren einer Reagenzzusammensetzung, die eine Mehrzahl von thiolselektiven polymeren Reagenzmolekülen umfasst, mit einer Zusammensetzung, die eine Mehrzahl von Butyrylcholinesterase-Dimermolekülen umfasst, unter Konjugationsbedingungen, um dadurch eine Konjugatmischung zu bilden, die monokonjugierte Butyrylcholinesterase-Dimere und dikonjugierte Butyrylcholinesterase-Dimere umfasst;
(b) Unterwerfen des Konjugatgemisches unter reduzierende Bedingungen, um ein reduziertes Gemisch zu bilden, das reduzierte nicht-konjugierte Butyrylcholinesterasemonomere und reduzierte monokonjugierte Butyrylcholinesterasemonomere umfasst;
(c) Abtrennen der reduzierten monokonjugierten Butyrylcholinesterase-Monomere von dem reduzierten Gemisch, um eine Zusammensetzung zu bilden, die reduzierte monokonjugierte Butyrylcholinesterase-Monomere umfasst; und
(d) Entfernen der reduzierenden Bedingungen aus der die reduzierten monokonjugierten Butyrylcholinesterase-Monomere umfassenden Zusammensetzung, um dadurch eine Zusammensetzung von dikonjugierten Butyrylcholinesterase-Dimeren zu bilden.

13. Verfahren nach Anspruch 12, bei dem die reduzierte monokonjugierte Butyrylcholinesterase-Monomere umfassende Zusammensetzung im Wesentlichen frei von reduzierten nichtkonjugierten Butyrylcholinesterase-Monomeren ist.

14. Verfahren nach Anspruch 12, bei dem das polymere Reagenz ein Poly(ethylenglykol)-Reagenz ist, das eine thiolreaktive funktionelle Gruppe trägt;
vorzugsweise das polymere Reagenz ein verzweigtes wasserlösliches Polymer mit der Struktur 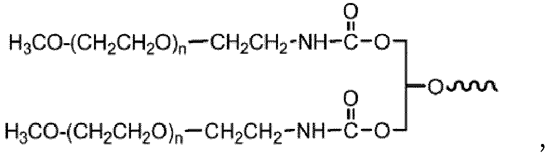 wobei jedes (n) unabhängig eine ganze Zahl mit einem Wert von etwa 2 bis 4000 ist; oder
mit der Struktur 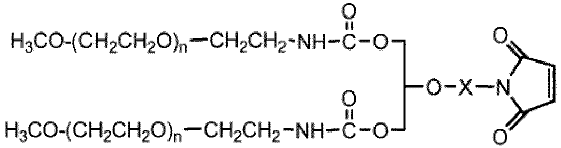 ist, wobei:
X eine ein oder mehrere Atome umfassende Spacer-Einheit ist; und
jedes (n) unabhängig eine ganze Zahl mit einem Wert von 2 bis 4000 ist.

15. Verfahren nach Anspruch 14, bei dem jedes Poly(ethylenglykol)-Reagenz ein gewichtsmittleres Molekulargewicht im Bereich von 5.000 bis etwa 100.000 Dalton aufweist;
vorzugsweise jedes Poly(ethylenglykol)-Reagenz ein gewichtsmittleres Molekulargewicht von 20.000 Dalton aufweist.

16. Verfahren nach Anspruch 12, bei dem das Unterwerfen der Zusammensetzung unter reduzierende Bedingungen die Zugabe eines Reduktionsmittels, ausgewählt aus 2-Mercaptoethanol, Dithiothreitol oder Tris(2-carboxyethyl)phosphin, zu dem Konjugatgemisch umfasst.

17. Verfahren nach Anspruch 16, bei dem das Entfernen des Reduktionsmittels das Entfernen des Reduktionsmittels durch Ionenaustauschchromatographie, Größenausschluss-Chromatographie oder Diafiltration umfasst.

## Revendications

1. Conjugué di-PEGylé comprenant deux polyéthylène-glycols rattachés de manière covalente à un dimère de butyrylcholinestérase dérivé de deux monomères butyrylcholinestérase séparés, chaque monomère butyrylcholinestérase ayant un seul polyéthylèneglycol rattaché de manière covalente au résidu de cystéine Cys66.

2. Conjugué selon la revendication 1, dans lequel la butyrylcholinestérase est préparée par recombinaison.

3. Conjugué selon la revendication 1 ou la revendication 2, dans lequel chaque polyéthylèneglycol est coiffé de façon terminale par un groupement de coiffage terminal choisi dans l'ensemble constitué par hydroxy, alcoxy, alcoxy substitué, alcénoxy, alcénoxy substitué, alcynoxy, alcynoxy substitué, aryloxy et aryloxy substitué.

4. Conjugué selon l'une quelconque des revendications 1 à 3, dans lequel chaque polyéthylèneglycol a une masse moléculaire moyenne en masse dans la plage allant d'environ 500 Daltons à environ 100 000 Daltons.

5. Conjugué selon la revendication 1, formé par réaction d'une butyrylcholinestérase avec un réactif polyéthylèneglycol portant un groupe maléimide et ayant la structure : 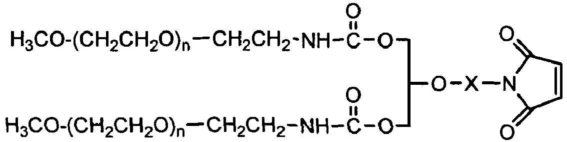 dans laquelle :
X est un groupement d'espacement constitué d'un ou plusieurs atomes ; et
chaque (n) est indépendamment un entier ayant une valeur d'environ 2 à environ 4 000.

6. Conjugué selon la revendication 5, dans lequel le réactif polymère portant un groupe maléimide a la structure suivante : 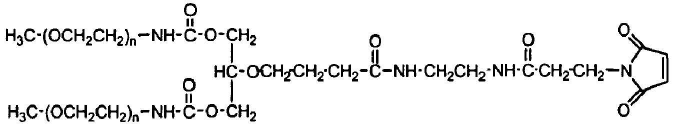 dans laquelle chaque (n) est indépendamment un entier ayant une valeur d'environ 225 à environ 1 930.

7. Conjugué selon la revendication 6, dans lequel chaque (n) est défini de façon à fournir -(OCH₂CH₂)- en ayant une masse moléculaire d'environ 20 kDa.

8. Conjugué selon la revendication 1, dans lequel le polyéthylèneglycol comprend la structure suivante : 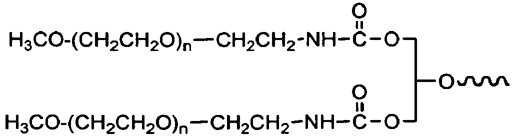 dans laquelle chaque (n) est indépendamment un entier ayant une valeur de 2 à 4 000.

9. Conjugué selon la revendication 1, dans lequel chaque monomère de butyrylcholinestérase dans le dimère a la structure suivante : 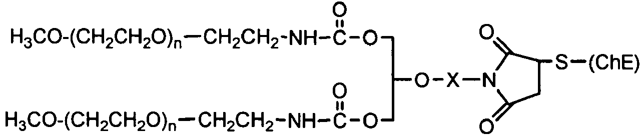 dans laquelle :
chaque (n) est indépendamment un entier ayant une valeur de 2 à 4 000 ;
X est un groupement d'espacement constitué d'un ou plusieurs atomes ; et
ChE est un résidu d'un monomère de butyrylcholinestérase, ou
dans lequel chaque monomère de butyrylcholinestérase dans le dimère a la structure suivante : 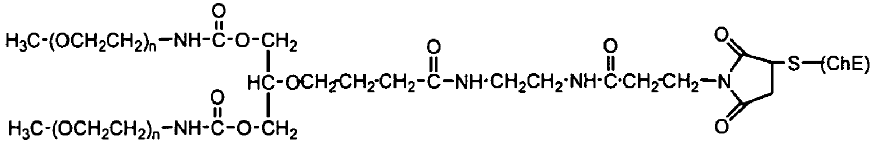 dans laquelle chaque (n) est indépendamment un entier ayant une valeur de 2 à 4 000.

10. Conjugué selon l'une quelconque des revendications 1 à 4, dans lequel le groupement butyrylcholinestérase est glycosylé.

11. Composition pharmaceutique comprenant un conjugué de l'une quelconque des revendications 1 à 10 et un excipient pharmaceutiquement acceptable.

12. Procédé de préparation d'un conjugué de dimère de dibutyrylcholinestérase diconjugué comprenant les étapes consistant à :
(a) combiner, dans des conditions de conjugaison, une composition de réactif comprenant une pluralité de molécules de réactif polymère thiol-sélectif avec une composition comprenant une pluralité de molécules de dimère de butyrylcholinestérase, pour former ainsi un mélange de conjugués comprenant des dimères de butyrylcholinestérase monoconjugués et des dimères de butyrylcholinestérase diconjugués ;
(b) soumettre le mélange de conjugués à des conditions réductrices pour former un mélange réduit comprenant des monomères de butyrylcholinestérase non conjugués réduits et des monomères de butyrylcholinestérase monoconjugués réduits ;
(c) séparer les monomères de butyrylcholinestérase monoconjugués du mélange réduit pour former une composition comprenant des monomères de butyrylcholinestérase monoconjugués réduits ; et
(d) retirer les conditions réductrices de la composition comprenant des monomères de butyrylcholinestérase monoconjugués réduits pour former ainsi une composition de dimères de butyrylcholinestérase diconjugués.

13. Procédé selon la revendication 12, dans lequel la composition comprenant des monomères de butyrylcholinestérase monoconjugués réduits est pratiquement exempte de monomères de butyrylcholinestérase non conjugués réduits.

14. Procédé selon la revendication 12, dans lequel le réactif polymère est un réactif polyéthylèneglycol portant un groupe fonctionnel thiol-réactif ;
de préférence le réactif polymère est un polymère soluble dans l'eau ramifié ayant la structure suivante : 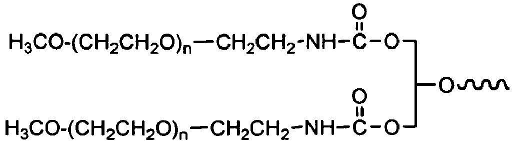 dans laquelle chaque (n) est indépendamment un entier ayant une valeur d'environ 2 à 4 000 ; ou
ayant la structure suivante : 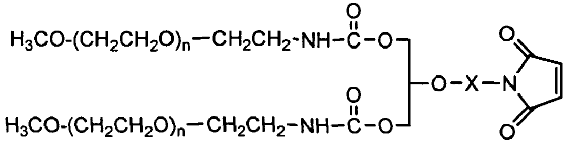 dans laquelle :
X est un groupement d'espacement constitué d'un ou plusieurs atomes ; et
chaque (n) est indépendamment un entier ayant une valeur de 2 à 4 000.

15. Procédé selon la revendication 14, dans lequel chaque réactif polyéthylèneglycol a une masse moléculaire moyenne en masse dans la plage d'environ 5 000 Daltons à environ 100 000 Daltons ;
de préférence chaque polyéthylèneglycol a une masse moléculaire moyenne en masse de 20 000 daltons.

16. Procédé selon la revendication 12, dans lequel l'étape consistant à soumettre la composition à des conditions réductrices comprend l'addition au mélange de conjugués d'un agent réducteur choisi parmi le 2-mercapto-éthanol, le dithiothréitol, et la tris (2-carboxyéthyl)phosphine.

17. Procédé selon la revendication 16, dans lequel l'étape consistant à retirer l'agent réducteur comprend le retrait de l'agent réducteur par chromatographie par échange d'ions, chromatographie d'exclusion diffusion, ou diafiltration.
